# EUROPEAN PATENT APPLICATION

(11) **EP 1 342 477 A1**
(43) Date of publication of application: **10.09.2003**
(21) Application number: 02025984.2
(22) Date of filing: 16.07.1997
(51) Int. Cl.: A61K 48/00, A61K 38/39, A61K 38/17, A61K 38/48, A61P 35/00

(54) **Cationic vehicle: dna complexes and their use in gene therapy**

(30) Priority: 16.07.1996 US 680845
(62) Divisional of application: 97112154.6
(71) Applicant: Mixson, Archibald James, M.D., Rockville, MD 20853 (US)
(72) Inventor: Mixson, Archibald James, M.D., Rockville, MD 20853 (US)
(74) Representative: Sieckmann, Ralf, Dr.

(57) **Abstract**

Cationic **vehicles:**DNA complexes comprising DNA encoding anti-angiogenic peptide or DNA encoding a tumor suppressor protein and DNA encoding an anti-angiogenic peptide, as well as their use in gene therapy, are disclosed.

SEQUENCE LISTING
(1) GENERAL INFORMATION:

(i) APPLICANT: Archibald James MIXSON

(ii) TITLE OF INVENTION: CATIONIC VEHICLE:DNA COMPLEXES
AND THEIR USE IN GENE THERAPY

(iii) NUMBER OF SEQUENCES: 25

(iv) CORRESPONDENCE ADDRESS:
(A) ADDRESSEE: COHAUSZ HASE DAWIDOWICZ
(B) STREET: Schumannstr. 97-99
(C) CITY: Düsseldorf
(D) STATE:
(E) COUNTRY: GERMANY
(F) ZIP: 40237

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE: July 16,1997
(C) CLASSIFICATION:

(viii) ATTORNEY/AGENT INFORMATION:
(A) NAME: SIECKAMNN, RALF, Dr.
(B) REGISTRATION NUMBER:
(C) REFERENCE/DOCKET NUMBER: 570414 (S/uc)

(ix) TELECOMMUNICATION INFORMATION:
(A) TELEPHONE: +49/2 11/9 14 60-0
(B) TELEFAX: +49/2 11/9 14 60-60

(2) INFORMATION FOR SEQ ID NO:1:

(i) SEQUENCE CHARACTERISTICS:
(A) LENGTH: 207 amino acids
(B) TYPE: amino acid
(D) TOPOLOGY: linear

(ii) MOLECULE TYPE: protein

(xi) SEQUENCE DESCRIPTION: SEQ ID NO:1:

Met Thr Glu Asn Lys Glu Leu Ala Asn Glu Leu Arg Arg Pro Pro Leu
1               5                   10                  15
Cys Tyr His Asn Gly Val Gln Tyr Arg Asn Asn Glu Glu Trp Thr Val
            20                  25                  30

Asp Ser Cys Thr Glu Cys His Cys Gln Asn Ser Val Thr Ile Cys Lys
        35                  40                  45

Lys Val Ser Cys Pro Ile Met Pro Cys Ser Asn Ala Thr Val Pro Asp
    45                  50                  55

Glu Glu Cys Cys Pro Arg Cys Trp Pro Ser Asp Ser Ala Asp Asp Gly
60                  65                  70                  75

Trp Ser Pro Trp Ser Glu Trp Thr Ser Cys Ser Thr Ser Cys Gly Asn
                75                  80                  85

Gly Ile Gln Gln Arg Gly Arg Ser Cys Asp Ser Leu Asn Asn Arg Cys
            90                  95                  100

Glu Gly Ser Ser Val Gln Thr Arg Thr Cys His Ile Gln Glu Cys Asp
        105                 110                 115

Lys Arg Phe Lys Gln Asp Gly Gly Trp Ser His Trp Ser Pro Trp Ser
    120                 125                 130

Ser Cys Val Thr Cys Gly Asp Gly Val Ile Thr Arg Ile Thr Leu Cys
135                 140                 145                 150

Asn Ser Pro Ser Pro Gln Met Asn Gly Lys Pro Cys Glu Gly Glu Ala
                155                 160                 165

Arg Glu Thr Lys Lys Ala Cys Lys Lys Asp Ala Cys Pro Ile Asn Glu
            170                 175                 180

Gly Trp Gly Pro Trp Ser Pro Trp Asp Ile Cys Ser Val Thr Cys Gly
        185                 190                 195

Gly Gly Val Gln Lys Arg Ser Arg Leu
    200                 205

(2) INFORMATION FOR SEQ ID NO:2:

(i) SEQUENCE CHARACTERISTICS:
(A) LENGTH: 656 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:2:

ATGACTGAAG AGAACAAAGA GTTGGCCAAT GAGCTGAGGC GGCCTCCCCT 50

ATGCTATCAC AACGGAGTTC AGTACAGAAA TAACGAGGAA TGGACTGTTG 100

ATAGCTGCAC TGAGTGTCAC TGTCAGAACT CAGTTACCAT CTGCAAAAAG 150

GTGTCCTGCC CCATCATGCC CTGCTCCAAT GCCACAGTTC CTGATGGAGA 200

ATGCTGTCCT CGCTGTTGGC CCAGCGACTC TGCGGACGAT GGCTGGTCTC 250

CATGGTCCGA GTGGACCTCC TGTTCTACGA GCTGTGGCAA TGGAATTCAG 300

CAGCGCGGCC GCTCCTGCGA TAGCCTCAAC AACCGATGTG AGGGCTCCTC 350

GGTCCAGACA CGGACCTGCC ACATTCAGGA GTGTGACAAA AGATTTAAAC 400

AGGATGGTGG CTGGAGCCAC TGGTCCCCGT GGTCATCTTG TTCTGTGACA 450

TGTGGTGATG GTGTGATCAC AAGGATCCGG CTCTGCAACT CTCCCAGCCC 500

CCAGATGAAT GGGAAACCCT GTGAAGGCGA AGCGCGGGAG ACCAAAGCCT 550

GCAAGAAAGA CGCCTGCCCC ATCAATGGAG GCTGGGGTCC TTGGTCACCA 600

TGGGACATCT GTTCTGTCAC CTGTGGAGGA GGGGTACAGA AACGTAGTCT 650

CTCTAA 656

(2) INFORMATION FOR SEQ ID NO:3:

(i) SEQUENCE CHARACTERISTICS:
(A) LENGTH: 434 amino acids
(B) TYPE: amino acid
(D) TOPOLOGY: linear

(ii) MOLECULE TYPE: protein

(xi) SEQUENCE DESCRIPTION: SEQ ID NO:3:

Met Thr Glu Asn Lys Glu Leu Ala Asn Glu Leu Arg Arg Pro Pro Leu
1               5                   10                  15

Cys Tyr His Asn Gly Val Gln Tyr Arg Asn Asn Glu Glu Trp Thr Val
            20                  25                  30

Asp Ser Cys Thr Glu Cys His Cys Gln Asn Ser Val Thr Ile Cys Lys
        35                  40                  45

Lys Val Ser Cys Pro Ile Met Pro Cys Ser Asn Ala Thr Val Pro Asp
    50                  55                  60

Glu Glu Cys Cys Pro Arg Cys Trp Pro Ser Asp Ser Ala Asp Asp Gly
65                  70                  75                  80

Trp Ser Pro Trp Ser Glu Trp Thr Ser Cys Ser Thr Ser Cys Gly Asn
                85                  90                  95

Gly Ile Gln Gln Arg Gly Arg Ser Cys Asp Ser Leu Asn Asn Arg Cys
            100                 105                 110

Glu Gly Ser Ser Val Gln Thr Arg Thr Cys His Ile Gln Glu Cys Asp
        115                 120                 125

Lys Arg Phe Lys Gln Asp Gly Gly Trp Ser His Trp Ser Pro Trp Ser
    130                 135                 140

Ser Cys Val Thr Cys Gly Asp Gly Val Ile Thr Arg Ile Thr Leu Cys
145                 150                 155                 160

Asn Ser Pro Ser Pro Gln Met Asn Gly Lys Pro Cys Glu Gly Glu Ala
                165                 170                 175

Arg Glu Thr Lys Lys Ala Cys Lys Lys Asp Ala Cys Pro Ile Asn Glu
            180                 185                 190

Gly Trp Gly Pro Trp Ser Pro Trp Asp Ile Cys Ser Val Thr Cys Gly
        195                 200                 205

Gly Gly Val Gln Lys Arg Ser Arg Leu Cys Val His Ser Arg Met Thr
    210                 215                 220

Glu Asn Lys Glu Leu Ala Asn Glu Leu Arg Arg Pro Pro Leu Cys Tyr
225                 230                 235                 240

His Asn Gly Val Gln Tyr Arg Asn Asn Glu Glu Trp Thr Val Asp Ser
                245                 250                 255

Cys Thr Glu Cys His Cys Gln Asn Ser Val Thr Ile Cys Lys Lys Val
            260                 265                 270

Ser Cys Pro Ile Met Pro Cys Ser Asn Ala Thr Val Pro Asp Glu Glu
        275                 280                 285

Cys Cys Pro Arg Cys Trp Pro Ser Asp Ser Ala Asp Asp Gly Trp Ser
    290                 295                 300

Pro Trp Ser Glu Trp Thr Ser Cys Ser Thr Ser Cys Gly Asn Gly Ile
305                 310                 315                 320

Gln Gln Arg Gly Arg Ser Cys Asp Ser Leu Asn Asn Arg Cys Glu Gly
                325                 330                 335

Ser Ser Val Gln Thr Arg Thr Cys His Ile Gln Glu Cys Asp Lys Arg
            340                 345                 350

Phe Lys Gln Asp Gly Gly Trp Ser His Trp Ser Pro Trp Ser Ser Cys
        355                 360                 365

Val Thr Cys Gly Asp Gly Val Ile Thr Arg Ile Thr Leu Cys Asn Ser
    370                 375                 380

Pro Ser Pro Gln Met Asn Gly Lys Pro Cys Glu Gly Glu Ala Arg Glu
385                 390                 395                 400

Thr Lys Lys Ala Cys Lys Lys Asp Ala Cys Pro Ile Asn Glu Gly Trp
                405                 410                 415

Gly Pro Trp Ser Pro Trp Asp Ile Cys Ser Val Thr Cys Gly Gly Gly
            420                 425                 430

Val Gln Lys Arg Ser Arg Leu
        435

(2) INFORMATION FOR SEQ ID NO:4:

(i) SEQUENCE CHARACTERISTICS:
(A) LENGTH: 1326 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:4:

ATGACTGAAG AGAACAAAGA GTTGGCCAAT GAGCTGAGGC GGCCTCCCCT 50

ATGCTATCAC AACGGAGTTC AGTACAGAAA TAACGAGGAA TGGACTGTTG 100

ATAGCTGCAC TGAGTGTCAC TGTCAGAACT CAGTTACCAT CTGCAAAAAG 150

GTGTCCTGCC CCATCATGCC CTGCTCCAAT GCCACAGTTC CTGATGGAGA 200

ATGCTGTCCT CGCTGTTGGC CCAGCGACTC TGCGGACGAT GGCTGGTCTC 250

CATGGTCCGA GTGGACCTCC TGTTCTACGA GCTGTGGCAA TGGAATTCAG 300

CAGCGCGGCC GCTCCTGCGA TAGCCTCAAC AACCGATGTG AGGGCTCCTC 350

GGTCCAGACA CGGACCTGCC ACATTCAGGA GTGTGACAAA AGATTTAAAC 400

AGGATGGTGG CTGGAGCCAC TGGTCCCCGT GGTCATCTTG TTCTGTGACA 450

TGTGGTGATG GTGTGATCAC AAGGATCCGG CTCTGCAACT CTCCCAGCCC 500

CCAGATGAAT GGGAAACCCT GTGAAGGCGA AGCGCGGGAG ACCAAAGCCT 550

GCAAGAAAGA CGCCTGCCCC ATCAATGGAG GCTGGGGTCC TTGGTCACCA 600

TGGGACATCT GTTCTGTCAC CTGTGGAGGA GGGGTACAGA AACGTAGTCG 650

TCTCTGCGTC GACTCTAGAA TGACTGAAGA GAACAAAGAG TTGGCCAATG 700

AGCTGAGGCG GCCTCCCCTA TGCTATCACA ACGGAGTTCA GTACAGAAAT 750

AACGAGGAAT GGACTGTTGA TAGCTGCACT GAGTGTCACT GTCAGAACTC 800

AGTTACCATC TGCAAAAAGG TGTCCTGCCC CATCATGCCC TGCTCCAATG 850

CCACAGTTCC TGATGGAGAA TGCTGTCCTC GCTGTTGGCC CAGCGACTCT 900

GCGGACGATG GCTGGTCTCC ATGGTCCGAG TGGACCTCCT GTTCTACGAG 950

CTGTGGCAAT GGAATTCAGC AGCGCGGCCG CTCCTGCGAT AGCCTCAACA 1000

ACCGATGTGA GGGCTCCTCG GTCCAGACAC GGACCTGCCA CATTCAGGAG 1050

TGTGACAAAA GATTTAAACA GGATGGTGGC TGGAGCCACT GGTCCCCGTG 1100

GTCATCTTGT TCTGTGACAT GTGGTGATGG TGTGATCACA AGGATCCGGC 1150

TCTGCAACTC TCCCAGCCCC CAGATGAATG GGAAACCCTG TGAAGGCGAA 1200

GCGCGGGAGA CCAAAGCCTG CAAGAAAGAC GCCTGCCCCA TCAATGGAGG 1250

CTGGGGTCCT TGGTCACCAT GGGACATCTG TTCTGTCACC TGTGGAGGAG 1300

GGGTACAGAA ACGTAGTCGT CTCTAA 1326

(2) INFORMATION FOR SEQ ID NO:5:

(i) SEQUENCE CHARACTERISTICS:
(A) LENGTH: 6 amino acids
(B) TYPE: amino acid
(D) TOPOLOGY: linear

(ii) MOLECULE TYPE: protein

(xi) SEQUENCE DESCRIPTION: SEQ ID NO:5:

Met Tyr Ile Gly Ser Arg 6
1               5
(2) INFORMATION FOR SEQ ID NO:6:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:6:

GTCGACATGT ATATTGGTTC TCGTTAAGTC GAC 33

(2) INFORMATION FOR SEQ ID NO:7:

(i) SEQUENCE CHARACTERISTICS:
(A) LENGTH: 25 amino acids
(B) TYPE: amino acid
(D) TOPOLOGY: linear

(ii) MOLECULE TYPE: protein

(xi) SEQUENCE DESCRIPTION: SEQ ID NO:7:

Met Tyr Ile Gly Ser Arg Gly Lys Ser Tyr Ile Gly Ser Arg
1               5                   10

Gly Lys Ser Tyr Ile Gly Ser Arg Gly Lys Ser
15                  20                  25

(2) INFORMATION FOR SEQ ID NO:8:

(i) SEQUENCE CHARACTERISTICS:
(A) LENGTH: 91 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:8:

GTCGACATGT ATATTGGTTC TCGTGTAAAA GATATATTGG TTCTCGTGG 50

TAAAAGATAT ATTGGTTCTC GTGGTAAAAG ATAAGTCGAC C 91

(2) INFORMATION FOR SEQ ID NO:9:

(i) SEQUENCE CHARACTERISTICS:
(A) LENGTH: 13 amino acids
(B) TYPE: amino acid
(D) TOPOLOGY: linear

(ii) MOLECULE TYPE: protein

(xi) SEQUENCE DESCRIPTION: SEQ ID NO:9:

Met Leu Tyr Lys Lys Ile Ile Lys Lys Leu Leu Glu Ser
1               5                   10

(2) INFORMATION FOR SEQ ID NO:10:

(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:10:

GTCGACATGC TTTATAAGAA GATCATCAAG AAGCTTCTTG AGAGTTAAGT 50

CGAC 54

(2) INFORMATION FOR SEQ ID NO:11:

(i) SEQUENCE CHARACTERISTICS:
(A) LENGTH: 46 amino acids
(B) TYPE: amino acids

(D) TOPOLOGY: linear

(ii) MOLECULE TYPE: protein

(xi) SEQUENCE DESCRIPTION: SEQ ID NO:11:

Met Leu Tyr Lys Lys Ile Ile Lys Lys Leu Leu Glu Ser Gly Lys Ser
1               5                   10                  15

Leu Tyr Lys Lys Ile Ile Lys Lys Leu Leu Glu Ser Gly Lys Ser Leu
            20                  25                  30

Tyr Lys Lys Ile Ile Lys Lys Leu Leu Glu Ser Gly Lys Ser
        35                  40                  45

(2) INFORMATION FOR SEQ ID NO:12:

(i) SEQUENCE CHARACTERISTICS:
(A) LENGTH: 153 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:12:

GTCGACATGC TTTATAAGAA GATCATCAAG AAGCTTCTTG AGAGTGGTAA     50

AAGACTTTAT AAGAAGATCA TCAAGAAGCT TCTTGAGAGT GGTAAAAGAC     100

TTTATAAGAA GATCATCAAG AAGCTTCTTG AGAGTGGTAA AAGATAAGTC     150

GAC                                                        153

(2) INFORMATION FOR SEQ ID NO:13:

(i) SEQUENCE CHARACTERISTICS:
(A) LENGTH: 9 amino acids
(B) TYPE: amino acid
(D) TOPOLOGY: linear

(ii) MOLECULE TYPE: protein

(xi) SEQUENCE DESCRIPTION: SEQ ID NO:13:

Met Phe Cys Tyr Trp Lys Val Cys Trp
1               5

(2) INFORMATION FOR SEQ ID NO:14:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:14:

GTCGACATGT TCTTGTATTG GAAGGGATTG TGGTAAGTCG AC 42

(2) INFORMATION FOR SEQ ID NO:15:

(i) SEQUENCE CHARACTERISTICS:
(A) LENGTH: 33 amino acids
(B) TYPE: amino acids
(D) TOPOLOGY: linear

(ii) MOLECULE TYPE: protein

(xi) SEQUENCE DESCRIPTION: SEQ ID NO:15:

Met Phe Cys Tyr Trp Lys Val Cys Trp Gly Lys Ser Phe Cys Tyr Trp
1               5                   10                  15

Lys Val Cys Trp Gly Lys Ser Phe Cys Trp Lys Val Cys Trp Gly Lys
            20                  25                  30

Ser

(2) INFORMATION FOR SEQ ID NO:16:

(i) SEQUENCE CHARACTERISTICS:
(A) LENGTH: 117 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:16:

GTCGACATGT TCTTGTATTG GAAGGGATTG TGGGGTAAAA GATTCTTGTA 50

TTGGAAGGGA TTGTGGGGTA AAAGATTCTT GTATTGGAAG GGATTGTGGG 100

GTAAAAGATA AGTCGAC 117

(2) INFORMATION FOR SEQ ID NO:17:

(i) SEQUENCE CHARACTERISTICS:
(A) LENGTH: 5 amino acids
(B) TYPE: amino acid
(D) TOPOLOGY: linear

(ii) MOLECULE TYPE: protein

(xi) SEQUENCE DESCRIPTION: SEQ ID NO:17:

Met Gly Arg Gly Asp
1               5

(2) INFORMATION FOR SEQ ID NO:18:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:18:

GTCGACATGT CTTTGTCTTG GAAGACTTTG ACTTAAGTCG AC 42

(2) INFORMATION FOR SEQ ID NO:19:

(i) SEQUENCE CHARACTERISTICS:
(A) LENGTH: 22 amino acids
(B) TYPE: amino acid
(D) TOPOLOGY: linear

(ii) MOLECULE TYPE: protein

(xi) SEQUENCE DESCRIPTION: SEQ ID NO:19:

Met Gly Arg Gly Asp Gly Lys Ser Gly Arg Gly Asp Gly Lys Ser Gly
1               5                   10                  15

Arg Gly Asp Gly Lys Ser
            20

(2) INFORMATION FOR SEQ ID NO:20:

(i) SEQUENCE CHARACTERISTICS:
(A) LENGTH: 81 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:20:

GTCGACATGG GTCGTGGTGA TGGTAAAAGA GGTCGTGGTG ATGGTAAAAG     50

AGGTCGTGGT GATGGTAAAA GATAAGTCGA C     81

(2) INFORMATION FOR SEQ ID NO:21:

i) SEQUENCE CHARACTERISTICS:
(A) LENGTH: 310 amino acids
(B) TYPE: amino acid
(D) TOPOLOGY: linear

(ii) MOLECULE TYPE: protein

(xi) SEQUENCE DESCRIPTION: SEQ ID NO:21:

Met Val Tyr Leu Ser Glu Cys Lys Thr Gly Ile Gly Asn Gly
1               5                   10

Tyr Arg Gly Thr Met Ser Arg Thr Lys Ser Gly Val Ala Cys
15                  20                  25

Gln Lys Trp Gly Ala Thr Phe Pro His Val Pro Asn Tyr Ser
    30                  35                  40

Pro Ser Thr His Pro Asn Glu Gly Leu Glu Glu Asn Tyr Cys
        45                  50                  55

Arg Asn Pro Asp Asn Asp Glu Gln Gly Pro Trp Cys Tyr Thr
            60                  65                  70

Thr Asp Pro Asp Lys Arg Try Asp Tyr Cys Asn Ile Pro Glu
                75                  80

Cys Glu Glu Glu Cys Met Tyr Cys Ser Gly Glu Lys Try Glu
85                  90                  95

Gly Lys Ile Ser Lys Thr Met Ser Gly Lys Asp Cys Gln Ala
    100                 105                 110

Trp Asp Ser Gln Ser Pro His Ala His Gly Tyr Ile Pro Ala
        115                 120                 125

Lys Phe Pro Ser Lys Asn Leu Lys Met Asn Tyr Cys His Asn
            130                 135                 140

Pro Asp Gly Glu Pro Arg Pro Trp Cys Phe Thr Thr Asp Pro
                145                 150

Thr Lys Arg Trp Glu Tyr Cys Asp Ile Pro Arg Cys Thr Thr
155                 160                 165

Pro Pro Pro Pro Pro Ser Pro Thr Tyr Gln Cys Leu Lys Gly
    170                 175                 180
Arg Gly Glu Asn Tyr Arg Gly Thr Val Ser Val Thr Val Ser
        185                 190                 195

Gly Lys Thr Cys Gln Arg Trp Ser Glu Gln Thr Pro His Arg
            200                 205                 210

His Asn Arg Thr Pro Glu Asn Phe Pro Cys Arg Asn Leu Glu
                215                 220

Glu Asn Tyr Cys Arg Asn Pro Asp Gly Glu Thr Ala Pro Trp
225                 230                 235

Cys Tyr Thr Thr Asp Ser Gln Leu Arg Trp Glu Tyr Cys Glu
    240                 245                 250

Ile Pro Ser Cys Glu Ser Ser Ala Ser Pro Asp Gln Ser Asp
        255                 260                 265

Ser Ser Val Pro Pro Glu Glu Gln Thr Pro Val Val Gln Glu
            270                275                  280

Cys Tyr Gln Ser Asp Gly Gln Ser Tyr Arg Gly Thr Ser Ser
                285                 290

Thr Thr Ile Thr Gly Lys Lys Cys Gln Ser Glu Gln Thr Pro
295                 300                 305

His Arg
    310

(2) INFORMATION FOR SEQ ID NO:22:

(i) SEQUENCE CHARACTERISTICS:
(A) LENGTH: 945 base pairs
(B) TYPE: nucleic acid
(C) STRANDEDNESS: single
(D) TOPOLOGY: linear

(ii) MOLECULE TYPE: DNA

(iii) HYPOTHETICAL: NO

GTCGACATGG TGTATCTGTC AGAATGTAAG ACCGGCATCG GCAACGGCTA CAGAGGAACC 60
ATGTCCAGGA CAAAGAGTGG TGTTGCCTGT CAAAAGTGGG GTGCCACGTT CCCCCACGTA 120
CCCAACTACT CTCCCAGTAC ACATCCCAAT GAGGGACTAG AAGAGAACTA CTGTAGGAAC 180
CCAGACAATG ATGAACAAGG GCCTTGGTGC TACACTACAG ATCCGGACAA GAGATATGAC 240
TACTGCAACA TTCCTGAATG TGAAGAGGAA TGCATGTACT GCAGTGGAGA AAAGTATGAG 300
GGCAAAATCT CCAAGACCAT GTCTGGACTT GACTGCCAGG CCTGGGATTC TCAGAGCCCA 360
CATGCTCATG GATACATCCC TGCCAAATTT CCAAGCAAGA ACCTGAAGAT GAATTATTGC 420
CACAACCCTG ACGGGGAGCC AAGGCCCTGG TGCTTCACAA CAGACCCCAC CAAACGCTGG 480
GAATACTGTG ACATCCCCCG CTGCACAACA CCCCCGCCCC CACCCAGCCC AACCTACCAA 540
TGTCTGAAAG GAAGAGGTGA AAATTACCGA GGGACCGTGT CTGTCACCGT GTCTGGGAAA 600
ACCTGTCAGC GCTGGAGTGA GCAAACCCCT CATAGGTGAG TCGAC 645

(2) INFORMATION FOR SEQ ID NO:23:
i) SEQUENCE CHARACTERISTICS:
(A) LENGTH: 623 amino acids
(B) TYPE: amino acid
(D) TOPOLOGY: linear

(ii) MOLECULE TYPE: protein

(xi) SEQUENCE DESCRIPTION: SEQ ID NO:23:

Met Val Tyr Leu Ser Glu Cys Lys Thr Gly Ile Gly Asn Gly
1               5                   10

Tyr Arg Gly Thr Met Ser Arg Thr Lys Ser Gly Val Ala Cys
15                  20                  25

Gln Lys Trp Gly Ala Thr Phe Pro His Val Pro Asn Tyr Ser
    30                  35                  40

Pro Ser Thr His Pro Asn Glu Gly Leu Glu Glu Asn Tyr Cys
        45                  50                  55

Arg Asn Pro Asp Asn Asp Glu Gln Gly Pro Trp Cys Tyr Thr
            60                  65                  70

Thr Asp Pro Asp Lys Arg Try Asp Tyr Cys Asn Ile Pro Glu
                75                  80

Cys Glu Glu Glu Cys Met Tyr Cys Ser Gly Glu Lys Try Glu
85                  90                  95

Gly Lys Ile Ser Lys Thr Met Ser Gly Lys Asp Cys Gln Ala
    100                 105                 110

Trp Asp Ser Gln Ser Pro His Ala His Gly Tyr Ile Pro Ala
        115                 120                 125

Lys Phe Pro Ser Lys Asn Leu Lys Met Asn Tyr Cys His Asn
            130                 135                 140

Pro Asp Gly Glu Pro Arg Pro Trp Cys Phe Thr Thr Asp Pro
                145                 150

Thr Lys Arg Trp Glu Tyr Cys Asp Ile Pro Arg Cys Thr Thr
155                 160                 165

Pro Pro Pro Pro Pro Ser Pro Thr Tyr Gln Cys Leu Lys Gly
    170                 175                 180

Arg Gly Glu Asn Tyr Arg Gly Thr Val Ser Val Thr Val Ser
        185                 190                 195

Gly Lys Thr Cys Gln Arg Trp Ser Glu Gln Thr Pro His Arg
            200                 205                 210

His Asn Arg Thr Pro Glu Asn Phe Pro Cys Arg Asn Leu Glu
                215                 220

Glu Asn Tyr Cys Arg Asn Pro Asp Gly Glu Thr Ala Pro Trp
225                 230                 235
Cys Tyr Thr Thr Asp Ser Gln Leu Arg Trp Glu Tyr Cys Glu
    240                 245                 250

Ile Pro Ser Cys Glu Ser Ser Ala Ser Pro Asp Gln Ser Asp
        255                 260                 265

Ser Ser Val Pro Pro Glu Glu Gln Thr Pro Val Val Gln Glu
            270                 275                 280

Cys Tyr Gln Ser Asp Gly Gln Ser Tyr Arg Gly Thr Ser Ser
                285                 290

Thr Thr Ile Thr Gly Lys Lys Cys Gln Ser Glu Gln Thr Pro
295                 300                 305

His Arg Gly Lys Ser Met Val Tyr Leu Ser Glu Cys Lys Thr
    310                 315                 320

Gly Ile Gly Asn Gly Tyr Arg Gly Thr Met Ser Arg Thr Lys
        325                 330                 335

Ser Gly Val Ala Cys Gln Lys Trp Gly Ala Thr Phe Pro His
            340                 345                 350

Val Pro Asn Tyr Ser Pro Ser Thr His Pro Asn Glu Gly Leu
                355                 360

Glu Glu Asn Tyr Cys Arg Asn Pro Asp Asn Asp Glu Gln Gly
365                 370                 375

Pro Trp Cys Tyr Thr Thr Asp Pro Asp Lys Arg Try Asp Tyr
    380                 385                 390

Cys Asn Ile Pro Glu Cys Glu Glu Glu Cys Met Tyr Cys Ser
        395                 400                 405

Gly Glu Lys Try Glu Gly Lys Ile Ser Lys Thr Met Ser Gly
            410                 415                 420

Lys Asp Cys Gln Ala Trp Asp Ser Gln Ser Pro His Ala His
                425                 430

Gly Tyr Ile Pro Ala Lys Phe Pro Ser Lys Asn Leu Lys Met
435                 440                 445

Asn Tyr Cys His Asn Pro Asp Gly Glu Pro Arg Pro Trp Cys
    450                 455                 460

Phe Thr Thr Asp Pro Thr Lys Arg Trp Glu Tyr Cys Asp Ile
        465                 470                 475

Pro Arg Cys Thr Thr Pro Pro Pro Pro Pro Ser Pro Thr Tyr
480 485 490
Gln Cys Leu Lys Gly Arg Gly Glu Asn Tyr Arg Gly Thr Val
495 500

Ser Val Thr Val Ser Gly Lys Thr Cys Gln Arg Trp Ser Glu
505 510 515

Gln Thr Pro His Arg His Asn Arg Thr Pro Glu Asn Phe Pro
520 525 530

Cys Arg Asn Leu Glu Glu Asn Tyr Cys Arg Asn Pro Asp Gly
535 540 545

Glu Thr Ala Pro Trp Cys Tyr Thr Thr Asp Ser Gln Leu Arg
550 555 560

Trp Glu Tyr Cys Glu Ile Pro Ser Cys Glu Ser Ser Ala Ser
565 570

Pro Asp Gln Ser Asp Ser Ser Val Pro Pro Glu Glu Gln Thr
575 580 585

Pro Val Val Gln Glu Cys Tyr Gln Ser Asp Gly Gln Ser Tyr
590 595 600

Arg Gly Thr Ser Ser Thr Thr Ile Thr Gly Lys Lys Cys Gln
605 610 615

Ser Glu Gln Thr Pro His Arg
620

(2) INFORMATION FOR SEQ ID NO:24:

(i) SEQUENCE CHARACTERISTICS:
(A) LENGTH: 1844 base pairs
(B) TYPE: nucleic acid
(C) STRANDEDNESS: single
(D) TOPOLOGY: linear

(ii) MOLECULE TYPE: DNA

(iii) HYPOTHETICAL: NO

GTCGACATGG TGTATCTGTC AGAATGTAAG ACCGGCATCG GCAACGGCTA CAGAGGAACC 60
ATGTCCAGGA CAAAGAGTGG TGTTGCCTGT CAAAAGTGGG GTGCCACGTT CCCCCACGTA 120
CCCAACTACT CTCCCAGTAC ACATCCCAAT GAGGGACTAG AAGAGAACTA CTGTAGGAAC 180
CCAGACAATG ATGAACAAGG GCCTTGGTGC TACACTACAG ATCCGGACAA GAGATATGAC 240
TACTGCAACA TTCCTGAATG TGAAGAGGAA TGCATGTACT GCAGTGGAGA AAAGTATGAG 300

GGCAAAATCT CCAAGACCAT GTCTGGACTT GACTGCCAGG CCTGGGATTC TCAGAGCCCA 360
CATGCTCATG GATACATCCC TGCCAAATTT CCAAGCAAGA ACCTGAAGAT GAATTATTGC 420
CACAACCCTG ACGGGGAGCC AAGGCCCTGG TGCTTCACAA CAGACCCCAC CAAACGCTGG 480
GAATACTGTG ACATCCCCCG CTGCACAACA CCCCCGCCCC CACCCAGCCC AACCTACCAA 540
TGTCTGAAAG GAAGAGGTGA AAATTACCGA GGGACCGTGT CTGTCACCGT GTCTGGGAAA 600
ACCTGTCAGC GCTGGAGTGA GCAAACCCCT CATAGGGGTA AAAGAATGGT GTATCTGTCA 660
GAATGTAAGA CCGGCATCGG CAACGGCTAC AGAGGAACCA TGTCCAGGAC AAAGAGTGGT 720
GTTGCCTGTC AAAAGTGGGG TGCCACGTTC CCCCACGTAC CCAACTACTC TCCCAGTACA 780
CATCCCAATG AGGGACTAGA AGAGAACTAC TGTAGGAACC CAGACAATGA TGAACAAGGG 840
CCTTGGTGCT ACACTACAGA TCCGGACAAG AGATATGACT ACTGCAACAT TCCTGAATGT 900
GAAGAGGAAT GCATGTACTG CAGTGGAGAA AAGTATGAGG GCAAAATCTC CAAGACCATG 960
TCTGGACTTG ACTGCCAGGC CTGGGATTCT CAGAGCCCAC ATGCTCATGG ATACATCCCT 1020
GCCAAATTTC CAAGCAAGAA CCTGAAGATG AATTATTGCC ACAACCCTGA CGGGGAGCCA 1080
AGGCCCTGGT GCTTCACAAC AGACCCCACC AAACGCTGGG AATACTGTGA CATCCCCCGC 1140
TGCACAACAC CCCCGCCCCC ACCCAGCCCA ACCTACCAAT GTCTGAAAGG AAGAGGTGAA 1200
AATTACCGAG GGACCGTGTC TGTCACCGTG TCTGGGAAAA CCTGTCAGCG CTGGAGTGAG 1260
CAAACCCCTC ATAGGTGAGT CGAC 1284

(2) INFORMATION FOR SEQ ID NO:25:

(i) SEQUENCE CHARACTERISTICS:
(A) LENGTH: 125 amino acids
(B) TYPE: amino acids
(D) TOPOLOGY: linear

(ii) MOLECULE TYPE: protein

(xi) SEQUENCE DESCRIPTION: SEQ ID NO:25:

Met Leu Pro Ile Cys Pro Gly Gly Ala Ala Arg Cys Gln Val
1               5                   10

Thr Leu Arg Glu Leu Phe Asp Arg Ala Val Val Leu Ser His
15                  20                  25

Tyr Ile His Asn Leu Ser Ser Glu Met Phe Ser Glu Phe Glu
30                  35                  40

Lys Arg Tyr Thr His Gly Arg Gly Phe Ile Thr Lys Ala Ile
    45                  50                  55

Asn Ser Cys His Thr Ser Ser Leu Ala Thr Pro Glu Asp Lys
            60                  65                  70

Glu Gln Ala Gln Gln Met Asn Gln Lys Asp Phe Leu Ser Leu
                75                  80

Ile Val Ser Ile Leu Arg Ser Trp Asn Glu Pro Leu Try His
85                  90                  95

Leu Val Thr Glu Val Arg Gly Met Gln Glu Ala Pro Gln Ala
    100                 105                 110

Ile Leu Ser Lys Ala Val Glu Ile Glu Glu Gln Thr Lys
        115                 120

(2) INFORMATION FOR SEQ ID NO:26:

(i) SEQUENCE CHARACTERISTICS:
(A) LENGTH: 390 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:26:

GTCGACATGT TGCCCATCTG TCCCGGCGGG GCTGCCCGAT GCCAGGTGAC CCTTCGAGAC 60
CTGTTTGACC GCGCCGTCGT CCTGTCCCAC TACATCCATA ACCTCTCCTC AGAAATGTTC 120
AGCGAATTCG ATAAACGGTA TACCCATGGC CGGGGGTTCA TTACCAAGGC CATCAACAGC 180
TGCCACACTT CTTCCCTTGC CACCCCCGAA GACAAGGAGC AAGCCCAACA GATGAATCAA 240
AAAGACTTTC TGAGCCTGAT AGTCAGCATA TTGCGATCCT GGAATGAGCC TCTGTATCAT 300
CTGGTCACGG AAGTACGTGG TATGCAAGAA GCCCCGGAGG CTATCCTATC CAAAGCTGTA 360
GAGATTGAGG AGCAAACCTA AGTCGAC 387

(2) INFORMATION FOR SEQ ID NO:27:

(i) SEQUENCE CHARACTERISTICS:
(A) LENGTH: 253 amino acids
(B) TYPE: amino acids
(D) TOPOLOGY: linear

(ii) MOLECULE TYPE: protein

(xi) SEQUENCE DESCRIPTION: SEQ ID NO:27:

Met Leu Pro Ile Cys Pro Gly Gly Ala Ala Arg Cys Gln Val
1               5                   10

Thr Leu Arg Glu Leu Phe Asp Arg Ala Val Val Leu Ser His
15                  20                  25

Tyr Ile His Asn Leu Ser Ser Glu Met Phe Ser Glu Phe Glu
    30                  35                      40

Lys Arg Tyr Thr His Gly Arg Gly Phe Ile Thr Lys Ala Ile
        45                  50                      55

Asn Ser Cys His Thr Ser Ser Leu Ala Thr Pro Glu Asp Lys
            60                  65                      70

Glu Gln Ala Gln Gln Met Asn Gln Lys Asp Phe Leu Ser Leu
                75                  80

Ile Val Ser Ile Leu Arg Ser Trp Asn Glu Pro Leu Try His
85                  90                  95

Leu Val Thr Glu Val Arg Gly Met Gln Glu Ala Pro Gln Ala
    100                 105                 110

Ile Leu Ser Lys Ala Val Glu Ile Glu Glu Gln Thr Lys Gly
        115                 120                 125

Lys Ser Met Leu Pro Ile Cys Pro Gly Gly Ala Ala Arg Cys
            130                 135                 140

Gln Val Thr Leu Arg Glu Leu Phe Asp Arg Ala Val Val Leu
                145                 150

Ser His Tyr Ile His Asn Leu Ser Ser Glu Met Phe Ser Glu
155                 160                 165

Phe Glu Lys Arg Tyr Thr His Gly Arg Gly Phe Ile Thr Lys
    170                 175                 180

Ala Ile Asn Ser Cys His Thr Ser Ser Leu Ala Thr Pro Glu
        185                 190                 195

Asp Lys Glu Gln Ala Gln Gln Met Asn Gln Lys Asp Phe Leu
200 205 210

Phe Leu Ser Leu Ile Val Ser Ile Leu Arg Ser Trp Asn Glu
215 220

Pro Leu Try His Leu Val Thr Glu Val Arg Gly Met Gln Glu
225 230 235

Ala Pro Gln Ala Ile Leu Ser Lys Ala Val Glu Ile Glu Glu
240 245 250

Gln Thr Lys
255

(2) INFORMATION FOR SEQ ID NO:28:

(i) SEQUENCE CHARACTERISTICS:
(A) LENGTH: 774 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:28:

ATGTTGCCCA TCTGTCCCGG CGGGGCTGCC CGATGCCAGG TGACCCTTCG AGACCTGTTT 60
GACCGCGCCG TCGTCCTGTC CCACTACATC CATAACCTCT CCTCAGAAAT GTTCAGCGAA 120
TTCGATAAAC GGTATACCCA TGGCCGGGGG TTCATTACCA AGGCCATCAA CAGCTGCCAC 180
ACTTCTTCCC TTGCCACCCC CGAAGACAAG GAGCAAGCCC AACAGATGAA TCAAAAAGAC 240
TTTCTGAGCC TGATAGTCAG CATATTGCGA TCCTGGAATG AGCCTCTGTA TCATCTGGTC 300
ACGGAAGTAC GTGGTATGCA AGAAGCCCCG GAGGCTATCC TATCCAAAGC TGTAGAGATT 360
GAGGAGCAAA CCGGTAAAAG AATGTTGCCC ATCTGTCCCG GCGGGGCTGC CCGATGCCAG 420
GTGACCCTTC GAGACCTGTT TGACCGCGCC GTCGTCCTGT CCCACTACAT CCATAACCTC 480
TCCTCAGAAA TGTTCAGCGA ATTCGATAAA CGGTATACCC ATGGCCGGGG GTTCATTACC 540
AAGGCCATCA ACAGCTGCCA CACTTCTTCC CTTGCCACCC CCGAAGACAA GGAGCAAGCC 600
CAACAGATGA ATCAAAAAGA CTTTCTGAGC CTGATAGTCA GCATATTGCG ATCCTGGAAT 660

GAGCCTCTGT ATCATCTGGT CACGGAAGTA CGTGGTATGC AAGAAGCCCC GGAGGCTATC 720
CTATCCAAAG CTGTAGAGAT TGAGGAGCAA ACCTAA 756

(2) INFORMATION FOR SEQ ID NO:29:

(i) SEQUENCE CHARACTERISTICS:
(A) LENGTH: 161 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:29:

ATGCTGAGGC GGCCTCCCCT ATGCTATCAC AACGGAGTTC AGTACAGAAA 50

TAACGGTAAA AGATCCCCGT GGTCATCTTG TTCTGTGACA TGTGGTGATG 100

GTGTGATGGT AAAAGAAGTG GTACCCTGTA GACAAGACAG TGGACACCTC 150

CTCCCCATTA A 161

(2) INFORMATION FOR SEQ ID NO:30:

(i) SEQUENCE CHARACTERISTICS:
(A) LENGTH: 63 amino acids
(B) TYPE: amino acid
(D) TOPOLOGY: linear

(ii) MOLECULE TYPE: protein

(xi) SEQUENCE DESCRIPTION: SEQ ID NO:30:

Met Leu Arg Arg Pro Pro Leu Cys Tyr His Asn Gly Val Gln Tyr Arg Asn
1               5                   10                  15

Asn Glu Glu Trp Thr Val Asp Ser Gly Lys Ser Ser Pro Trp Ser Ser Cys
        20                  25                  30

Ser Val Thr Cys Gly Asp Gly Val Ile Thr Arg Ile Gly Lys Ser Ser Pro
35                  40                  45                  50

Trp Asp Ile Cys Ser Val Thr Cys Gly Gly Gly Val
            55                  60

(2) INFORMATION FOR SEQ ID NO:31:

(i) SEQUENCE CHARACTERISTICS:
(A) LENGTH: 185 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:31:

ATGCTGAGGC GGCCTCCCCT ATGCTATCAC AACGGAGTTC AGTACAGAAA 50

TAACGGTAAA AGATCCCCGT GGTCATCTTG TTCTGTGACA TGTGGTGATG 100

GTGTGATGGT AAAAGAAGTG GTACCCTGTA GACAAGACAG TGGACACCTC 150

CTCCCCATTA TATTGGTTCT CGTGGTAAAA GATAA 185

(2) INFORMATION FOR SEQ ID NO:32:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:32:

TAGGTCTAGA ATGACTGAAG AGAACAAAGA G 31

(2) INFORMATION FOR SEQ ID NO:33:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA

(iii) HYPOTHETICAL: NO

(xi) SEQUENCE DESCRIPTION: SEQ ID NO:33:

ATGGTCTAGA TTAGAGACGA CTACGTTTCT G 31

## Description

### FIELD OF THE INVENTION

The present invention relates to cationic vehicles:DNA complexes (*i.e.* cationic liposome:DNA complexes, cationic polymer:DNA complexes) comprising DNA encoding an anti-angiogenic peptide, or DNA encoding a tumor suppressor protein and DNA encoding an anti-angiogenic peptide, and their use in gene therapy.

### BACKGROUND OF THE INVENTION

### I. Gene Therapy

Development of gene therapy techniques is approaching clinical realization for the treatment of neoplastic and metabolic diseases. The main obstacle in the treatment of malignant diseases, however, remains in the vector delivery system of the transgene to a distant target tissue.

Vectors carrying genes are commonly divided into viral and non-viral vector categories. Unfortunately, all vectors described to date have significant limitations. For example, replication-deficient retroviral vectors can efficiently transfect dividing cells. Local intratumoral injection of retroviruses that contain a thymidine kinase transgene has been used successfully to affect regression of gliomas (Culver et al, *Science,* 256:1550-1552 (1992)). However, retroviruses have the potential to cause insertional mutagenesis. As a result, their use has been limited to either direct injection of tumors or to *ex vivo* gene transfer trials. Unlike retroviral vectors, adenoviral vectors can also transfect non-dividing cells, and their ability to cause insertional mutagenesis is greatly reduced. However, they have the undesirable potential to activate the immune system in humans (Crystal, *Science,* 270:404-410, (1995). Attempts are underway to minimize the immunogenicity of the adenoviral vectors, but the potential toxicity of viral vectors will most likely limit their use for systemic delivery of genes in the near future.

Non-viral vectors of DNA include liposomes, peptides, proteins and polymers (Ledley, *Current Opinion in Biotechnology,* 5:626-636 (1994)). Of these, liposomes are the most commonly used non-viral vectors of DNA. The major advantage of liposomes over retroviruses is that DNA is not incorporated into the genome, and unlike adenoviral vectors, they are not immunogenic. However, the major limitation of liposomes is that they are not as efficient as viral vectors in transfecting many cell types. Until recently, their medical utility was limited by their rapid uptake by phagocytic cells. Interest in liposomes as a vector was rejuvenated by two technological advances that have produced a renaissance in the field. First, stearically stabilized (Stealth) liposomes represent a significant breakthrough in that they are non-reactive, and are not readily taken up by the reticuloendothelial system (RES). Stealth liposomes are composed of lipids rich in oxygen in their head group (ethylene glycol or glycolipids) which provide a stearic barrier outside of the membrane. As a result, Stealth liposomes remain in the blood for up to 100 times longer than conventional liposomes, and can thus increase pharmacological efficacy (Papahajopoulos, *In: Stealth liposomes,* Ed., Lasic et al, CRC Press (1995); and Lasic et al, *Science,* 267:1275-76 (1995)). However, Stealth liposomes are still not particularly efficient in transfection of cells or as vectors for DNA.

The second significant advance in liposome technology has been the use of cationic liposomes complexed to negatively-charged DNA. Cationic liposomes can condense DNA, and increase transfection yields several orders of magnitude. In the cationic liposome:DNA complex, the nucleic acids or oligonucleotides are not encapsulated, but are simply complexed with small unilamellar vesicles by electrostatic interactions. The exact nature of the cationic liposome:DNA complex is not clear, but intricate topological rearrangements of the cationic liposome:DNA complex occur, including DNA condensation, liposome aggregation, and fusion. This supramolecular complex can be added to cells *in vitro,* injected parenterally, or aerosolized for pulmonary applications (Lasic et al, *Science,* 267:1275-1276 (1995)). Further, the intravenous injection into mice of high concentrations of the CAT gene (100 µg or greater) complexed with cationic liposomes has been found to result in 40% transfection efficiency of well vascularized tissues, such as the spleen (Zhu et al, *Science,* 261:209-211 (1993)). However, a major challenge of gene therapy remains the systemic delivery of transgenes to the tumor or peritumoral area that will effectively decrease the size of primary tumors and their metastases. This is because unlike the spleen and bone marrow, which are highly vascular and have a high capacity to filter macromolecules from the blood stream, most organs and tumors do not have this capacity, and the transfection efficiency of these tissues with liposomes is low (Marshall, *Science,* 269:1051-1055 (1995)). In addition, another limitation of cationic liposome: DNA complexes is that their 1/2 life in the blood stream is less than one hour (Allen et al, In: *Liposome* *Technology*-Vol. III, Ed., Gregoriadis G et al, CRC Press (1993)). Sufficient transfection of the target cell by vectors carrying therapeutic genes has thus far been the rate-limiting step in gene therapy.

### II. Tumor Suppressor Genes

Tumor suppressor genes are well-known in the art, and include the p53 gene (Baker et al, *Science,* 249:912-915 (1990)), the p21 gene (El-Deiry et al, *Cell,* 75:817-825 (1993); and Harper et al, *Cell,* 75:805-816 (1993)), and the rb gene (Bookstein et al, *Science,* 247:712-715 (1990)).

Mutations in the tumor suppressor gene p53 are known to occur in over 50% of human tumors, including metastatic breast cancer (Vogelstein, *Nature,* 348:681-682 (1990)). Breast cancer is one of the leading causes of death in women in North America and Western Europe, affecting nearly 10% of this population living to 80 years of age, and one million new cases are predicted by the end of this decade (Miller et al, *Int. J. Cancer*, 37:173-177 (1986)). Although the molecular basis of multistage carcinogenesis in breast cancer is not well understood, the metastatic potential of breast cancers has been correlated with the presence of point mutations in the p53 gene (Wang et al, *Oncogene,* 8:279-288 (1993)). Various groups have found that reintroduction of the wild-type P53 into a tumor cell has the therapeutic potential to inactivate the proliferative effects of the mutated product (Bookstein et al, *Cancer,* 71:1179-1186 (1993); Chen et al, *Science*, 250:1576-1580 (1990); and Baker et al, *Science,* 249:912-915 (1990)). For example, *in vitro* transfection and retroviral-mediated transfer of a single copy of the p53 transgene into a variety of tumor cells, including breast cancer cells, was found to result in a decrease in growth rate and/or attenuated tumor development once those transfected cells were implanted into nude mice (Wang et al, *Oncogene,* 8:279-288 (1993); Baker et al, *Science,* 249:912-915 (1990)); Bookstein et al, *Science,* 247:712-715 (1990); Cheng et al, *Cancer Res*., 52:222-226 (1992); Isaacs et al, *Cancer Res*., 51:4716-4720 (1991); Diller et al, *Mol. Cell. Biol*., 10:5772-5781 (1990); Chen et al, *Oncogene*, 6:1799-1805 (1991); and Zou et al, *Science*, 263:526-529 (1994)). In addition, intratracheal injection of a retrovirus containing the p53 transgene has been shown to significantly inhibit the growth of lung tumors (Fujiwara et al, *J. Natl. Cancer. Inst*., 86:1458-1462 (1994)). Further, systemic intravenous administration of a β-actin promoter-containing vector containing the p53 coding sequence complexed to cationic liposomes has been found to affect the tumor growth of a malignant line of breast cancer cells injected into nude mice (Lesoon-Wood et al, *Proc. Am. Ass. Cancer Res.,* 36:421 (1995); and Lesoon-Wood et al, *Human Gene Ther.,* 6:39-406 (1995)). Of the 15 tumors treated in this study, four of these tumors did not respond to treatment. Because of the unresponsiveness of these tumors, new therapies were sought in the present invention to more effectively decrease the size of these tumors. Based on the *in vitro* data concerning p53, one might expect that p53 decreases the size of the tumors due to efficient transfection of the tumor. However, less than 5% of the tumor was transfected after three injections of a cationic liposome:marker (CAT) gene. Furthermore, some endothelial cells of the tumor were transfected with this marker gene. Thus, the primary target of cationic liposome:p53 complex may be the vasculature system of the tumor. Given that angiogenesis is critical for the development of any human tumor, as well as for metastastases (Fidler et al, *Cell,* 79:185-188 (1994)), this therapy should be widely applicable to a wide variety of tumors.

p53 coordinates multiple responses to DNA damage. DNA damage results in an increase in the level of the p53 protein. Following DNA damage, an important function of wild-type p53 function is to control the progression of cells from G1 to S phase. Recently, several groups have found that p53 transcriptionally activates a p21 kd protein (also known as WAF1 or Cip1), an inhibitor of cyclin-dependent kinases (CDKs) (El-Deiry et al, *supra;* and Harper et al, *supra).* Inhibition of CDK activity is thought to block the release of the transcription factor E2F, and related transcription factors from the retinoblastoma protein RB, with consequent failure to activate transcription of genes required for S phase entry (Harper et al, *supra;* and Xiong et al, *Nature,* 366:701-704 (1993)). Evidence consistent with the model that pRb is a downstream effector of p53-induced G1 arrest has recently been reported (Dulic et al, *Cell*, 76:1013-1023 (1994)). Thus, p53 regulates cell cycle through two proteins: p21 and rb.

### III. Anti-Angiogenic Proteins

Proteins with anti-angiogenic activities are well-known and include: thrombospondin I (Kosfeld et al, *J. Biol. Chem*., 267:16230-16236 (1993); Tolsma et al, *J. Cell Biol.,* 122:497-511 (1993); and Dameron et al, *Science,* 265:1582-1584 (1995)), IL-12 (Voest et al, *J. Natl. Cancer Inst.,* 87:581-586 (1995)), protamine (Ingber et al, *Nature,* 348:555-557 (1990)), angiostatin (O'Reilly et al, *Cell*, 79:315-328 (1994)), laminin (Sakamoto et al, *Cancer Res.,* 5:903-906 (1991)), and a prolactin fragment (Clapp et al, *Endocrinol.,* 133:1292-1299 (1993)). In addition, several anti-angiogenic peptides have been isolated from these proteins (Maione et al, *Science*, 247:77-79 (1990); Woltering et al, *J. Surg. Res.*, 50:245-251 (1991); and Eijan et al, *Mol. Biother.*, 3:38-40 (1991)).

Thrombospondin I (hereinafter "TSPI") is a large trimeric glycoprotein composed of three identical 180 kd subunits (Lahav et al, *Semin. Thromb. Hemostasis*, 13:352-360 (1987)) linked by disulfide bonds (Lawer et al, *J. Cell Biol*., 103:1635-1648 (1986); and Lahav et al, *Eur. J. Biochem*., 145:151-156 (1984)). The majority of anti-angiogenic activity is found in the central stalk region of this protein (Tolsma et al, *supra).* There are at least two different structural domains within this central stalk region that inhibit neovascularization (Tolsma et al, *supra).*

Besides TSPI, there are five other proteins (fibronectin, laminin, platelet factor-4, angiostatin, and prolactin fragment) in which peptides have been isolated that inhibit angiogenesis. In addition, analogues of the peptide somatostatin are known to inhibit angiogenesis.

Fibronectin (FN) is a major surface component of many normal cells, as well as a potent dell spreading factor. During transformation, the loss of cellular FN has been observed. Furthermore, the addition of fibronectin to transformed cells restores the normal phenotype. It has been found that either heparin-binding or cell-adhesion fragments from FN can inhibit experimental metastasis, suggesting that cell surface proteolyglycans are important in mediating the adhesion of metastatic tumor cells (McCarthy et al, *J. Natl. Cancer Inst*., 80:108-116 (1988)). It has also been found that FN and one of its peptides inhibits *in vivo* angiogenesis (Eijan et al, *Mol. Biother*., 3:38-40 (1991)).

Laminin is a major component of the basement membrane, and is known to have several biologically active sites that bind to endothelial and tumor cells. Laminin is a cruciform molecule that is composed of three chains, an A Chain and two B chains. Several sites in laminin have been identified as cell binding domains. These sites promote cellular activities *in vitro,* such as cell spreading, migration, and cell differentiation. Two peptides from two sites of the laminin B1 chain are known to inhibit angiogenesis (Grant et al, *Path. Res. Pract*., 190:854-863 (1994)).

Platelet factor-4 (PF4) is a platelet α-granule protein originally characterized by its high affinity for heparin. The protein is released from platelets during aggregation as a high molecular weight complex of a tetramer of the PF4 polypeptide and chondroitin sulfate, which dissociates at high ionic strength. PF4 has several biological properties including immunosuppression, chemotactic activity for neutrophils and monocytes as well as for fibroblasts, inhibition of bone resorption, and inhibition of angiogenesis. The angiostatic properties of human PF4 are associated with the carboxyl-terminal, heparin binding region of the molecule. A 12 amino acid synthetic peptide derived therefrom has been discovered to have marked angiostatic affects (Maione et al, *Science,* 247:77-79 (1990)).

Although somatostatin is not a protein, it is a naturally-occurring cyclic 14 amino acid peptide whose most-recognized function is the inhibition of growth hormone (GH) secretion. Somatostatin is widely distributed in the brain, in which it fulfills a neuromodulatory role, and in several organs of the gastrointestinal tract, where it can act as a paracrine factor or as a true circulating factor. The role played by the neuropeptide somatostatin, also known as somatotropin release inhibitory factor (SRIF), in human cancer is not well understood. Recent investigations involving somatostatin receptors in normal and neoplastic human tissues suggest that the action is complex, and involves both direct and indirect mechanisms. One of the anti-tumor mechanisms of these synthetic somatostatin analogues may be an anti-angiogenic effect (Woltering et al, *J. Surg. Res*., 50:245-50 (1990)). In a recent study, the ability of native somatostatin and nine somatostatin analogues to inhibit angiogenesis were evaluated. The most potent somatostatin analogue was found to be approximately twice as potent as the naturally-occurring somatostatin (Barrie et al, *J. Surg. Res*., 55:446-50 (1993)).

Angiostatin is a 38 kDa polypeptide fragment of plasminogen. Whereas plasminogen has no fibrinogenic activity, angiostatin has marked angiogenic activity (O'Rielly MS, et al Cell, 79:315-28 (1994)). Angiostatin was isolated when it was observed that the primary tumor suppresed metastases. That is, when the primary tumor was removed, the metastases grew. Admininistration of angiostatin blocks neovascularization and growth of metastases.

Finally, a 16kd fragment of prolactin has been found to be angiogenic. Similar to plasminogen, prolactin is not anti-angiogenic but the prolactin fragment is a potent in vivo and in vitro inhibitor of angiogenesis (Clapp C. et al. Endocrinology. 133:1292-1299 (1993).

Despite the evidence that anti-angiogenic peptides are effective anti-tumor agents, as well as the great deal of interest in targeting genes toward the vasculature, there have been no published reports on effective *in vivo* gene therapy regimens with established anti-angiogenic DNA sequences.

There are several reasons why gene therapy utilizing antiangiogenic genes have not been used or why antiangiogenic peptides are effective and the liposome: antiangiogenic gene may not be. First, there are significant physical differences between the liposome: DNA complexes and their peptides. Cationic liposomes have a 1/2 life of less than one hour (Allen TM and Papahajopoulos D, In: Liposome Technology-Vol. III, Ed., Gregoriadis G et al, CRC Press (1993)). whereas the most effective of the antiangiogenic peptides (i.e angiostatin) have a 1/2 life of two days (Folkman J, The John Krantz, Jr Lecture in Pharmacology, UMAB, 4/30/96). Since cationic liposomes form large aggregates when mixed with DNA, the distribution of these complexes is likely to be quited different from the much smaller peptides (need reference). These properties of the liposomes may account for the low transfection efficiency of a tumor. Therefore, it is uncertain as to whether these liposome:DNA complexes will reach their cellular targets.

Furthermore, the exact receptor target or mechanisms of these antiangiogenic peptides are unknown (Tolsma et al, supra). For example, it is unknown whether these receptor targets are intracellular or extracellular. The anti-angiogenic genes that are complexed to liposomes encode their respective proteins inside the cell, and proteins without secretory sequences remain inside the cell. Thus, it is unclear that a intracellular antiangiogenic peptide derived from a systemically transfected gene will reach its cellullar and/or receptor target.

The only transfected antiangiogenic gene that has inhibited tumor growth is full length thrombospondin I. In this study *(Weinstat-Saslow et al, Cancer Research 54, 6504-6511, (1994*)) tumor cells that expressed 15 fold higher levels of the thrombospondin I in vitro than baseline cells were implanted into the mice. This transfected full length thrombospondin I was secreted from the tumor cells to inhibit angiogeneis, and effectively reduced the tumor by 60%. Thus, this study determined that transfection of 100% of the tumor cells with a highly expressed secreted antiangiogenic gene was able to reduce tumor size.

### SUMMARY OF THE INVENTION

An object of the present invention is to provide cationic vehicles:DNA complexes, such as liposome complexes containing DNA encoding anti-angiogenic peptides or cationic complexes containing DNA encoding an anti-angiogenic peptides.

Another object of the present invention is to provide a method of anti-angiogenic gene therapy.

Still another object of the present invention is to provide liposome complexes containing DNA encoding an anti-angiogenic peptide.

Yet another object of the present invention is to provide liposome complexes containing DNA encoding an anti-angiogenic peptide in combination with DNA encoding a tumor suppressor gene.

A further object of the present invention is to provide liposome complexes containing DNA encoding concatamers of the same or different anti-angiogenic peptides.

An additional object of the present invention is to provide a method for inhibiting tumor growth in a subject, or alternatively, to use these complexes for the production of a medicament, especially for inhibiting tumor growth in a subject.

These and other objects of the present invention, which will be apparent from the detailed description of the invention provided hereinafter, have been met in one embodiment, by a cationic liposome:DNA complex comprising DNA encoding an anti-angiogenic peptide and DNA encoding a tumor suppressor protein.

Further objects of the present invention were solved by the features defined in the present set of claims, but especially the preferred embodiments defined in claims 2 to 25.

Based on the present invention, it is anticipated that one skilled in the gene therapy could utilize other cationic carriers (polylysine, polyhistidine, polycat57, Superfect, and polyethyleneimine) complexed with the antiangiogenic genes to inhibit tumors.

### DETAILED DESCRIPTION OF THE INVENTION

As discussed above, in one embodiment, the above-described objects of the present invention have been met by a non-viral:DNA complex comprising DNA encoding an anti-angiogenic peptide and DNA encoding a tumor suppressor protein.

The particular non-viral carrier (liposomes-neutral or non-cationic, see below) , polyethylenimine Polycat57 (Avanti Lipids), polylysine (Sigma), polyhistidine (Sigma) , Superfect (Qiagen), are not criti-cal to the present invention although cationic lipo-somes are preferable carriers. Examples of cationic lipids which can be employed in the present invention include 1,2-dioleolyl-sn-glycero-3-ethylphosphocholine (Avanti, Birmingham, AL), 1,2-dimyristoyl-sn-glycero-3-ethylphosphocholine (Avanti, Birmingham, AL), and (2,3-diol-eyloxy)propyl-N,N,N-trimethylammonium chloride (DOTMA) (Syntex Corp., Palo Alto, CA).
The cationic lipids are preferably used in a mixture with dioleoylphophatidylethanolamine (DOPE) (Avanti, Bimingham, AL). In the present invention, the amount of cationic lipid present in the mixture is generally in the range of from 100% to 40% (w/w), preferably about 50% (w/w); and the amount of DOPE present in the mixture is generally in the range of from 0% to 60% (w/w), preferably about 50% (w/w); and the amount of pegylated lipid (1,2 -diacyl-sn-glycero-3-phosphoethanolamine-N-[Poly(ethylene glycol) 2000] present in the mixture is generally in the range of from 0% to 10% (w/w), preferably about 1% (w/w).

The particular ligand will be dependendent on the the tumor/peritumoral targeted. Examples of targets on tumors include Her2 (breast), CEA (colon), ferritin receptor (breast, lung, and ovary), and the tumor vasculature (αvB3 integrins or tissue factor). Antibodies directed toward Her2, CEA, and the tumor's vasculature will be coupled to 1% of the pegylated lipid hydroxyl group of the pegylated lipid with a water soluble carbodiimide (1-ethyl-3 (3-dimethylamino-propyl) carbodiimide), and purified over a sepharose CL-6B column. Similarly, ligands to the tumor (ferritin) and/or the vasculature (the peptide, RGD) are covalently attached to the hydroxyl the pegylated lipids.

The particular tumor suppressor gene employed is not critical to the present invention. Examples of such tumor suppressor genes include the p53 gene, the p21 gene (El-Deiry et al, *supra;* and Harper, *supra),* and the rb gene (Bookstein et al, *supra).* The p53 gene is the preferred tumor suppressor gene employed in the present invention.

The particular anti-angiogenic peptide encoded by the DNA is not critical to the present invention.

Examples of said peptides include a fragment of thrombospondin I (TSPf) having the following amino acid sequence (the amino acid sequences that are known to be anti-angiogenic are underlined): 
MTEENKELANELRRPPLCYHNGVQYRNNEEWTVDSCTECHCQNSVTICKKVSC
PIMPCSNATVPDGECCPRCWPSDSADDGWSPWSEWTSCSTSCGNGIQQRGRSC
DSLNNRCEGSSVQTRTCHIQECDKRFKQDGGWSHWSPWSSCSVTCGDGVITRI
TLCNSPSPQMNGKPCEGEARETKACKKDACPINGGWGPWSPWDICSVTCGGGV
QKRSRL (SEQ ID NO:1),
 which is encoded by the following DNA sequence (nucleotides 1013-1650 of the TSPI gene; the underlined sequences encode the anti-angiogenic peptides; the bold TAA is the stop codon): 
ATGACTGAAGAGAACAAAGAGTTGGCCAATGAGCTGAGGCGGCCTCCCCT
ATGCTATCACAACGGAGTTCAGTACAGAAATAACGAGGAATGGACTGTTG
ATAGCTGCACTGAGTGTCACTGTCAGAACTCAGTTACCATCTGCAAAAAG
GTGTCCTGCCCCATCATGCCCTGCTCCAATGCCACAGTTCCTGATGGAGA
ATGCTGTCCTCGCTGTTGGCCCAGCGACTCTGCGGACGATGGCTGGTCTC
CATGGTCCGAGTGGACCTCCTGTTCTACGAGCTGTGGCAATGGAATTCAG
CAGCGCGGCCGCTCCTGCGATAGCCTCAACAACCGATGTGAGGGCTCCTC
GGTCCAGACACGGACCTGCCACATTCAGGAGTGTGACAAAAGATTTAAAC
AGGATGGTGGCTGGAGCCACTGGTCCCCGTGGTCATCTTGTTCTGTGACA
TGTGGTGATGGTGTGATCACAAGGATCCGGCTCTGCAACTCTCCCAGCCC
 
CCAGATGAATGGGAAACCCTGTGAAGGCGAAGCGCGGAGACCAAAGCCT
GCAAGAAAGACGCCTGCCCCATCAATGGAGGCTGGGGTCCTTGGTCACCA
TGGGACATCTGTTCTGTCACCTGTGGAGGAGGGGTACAGAAACGTAGTCT
CTCTAA (SEQ ID NO:2);
 a concatamer of TSPf having the following amino acid sequence (the intervening sequence is underlined): 
MTEENKELANELRRPPLCYHNGVQYRNNEEWTVDSCTECHCQNSVTICKKVSC
PIMPCSNATVPDGECCPRCWPSDSADDGWSPWSEWTSCSTSCGNGIQQRGRSC
DSLNNRCEGSSVQTRTCHIQECDKRFKQDGGWSHWSPWSSCSVTCGDGVITRI
TLCNSPSPQMNGKPCEGEARETKACKKDACPINGGWGPWSPWDICSVTCGGGV
QKRSRLCVHSRMTEENKELANELRRPPLCYHNGVQYRNNEEWTVDSCTECHCQ
NSVTICKKVSCPIMPCSNATVPDGECCPRCWPSDSADDGWSPWSEWTSCSTSC
GNGIQQRGRSCDSLNNRCEGSSVQTRTCHIQECDKRFKQDGGWSHWSPWSSCS
VTCGDGVITRITLCNSPSPQMNGKPCEGEARETKACKKDACPINGGWGPWSPW
DICSVTCGGGVQKRSRL (SEQ ID NO:3),
 which is encoded by the following DNA sequence (the intervening sequence is underlined): 
ATGACTGAAGAGAACAAAGAGTTGGCCAATGAGCTGAGGCGGCCTCCCC
TATGCTATCACAACGGAGTTCAGTACAGAAATAACGAGGAATGGACTGTT
GATAGCTGCACTGAGTGTCACTGTCAGAACTCAGTTACCATCTGCAAAAA
GGTGTCCTGCCCCATCATGCCCTGCTCCAATGCCACAGTTCCTGATGGAG
AATGCTGTCCTCGCTGTTGGCCCAGCGACTCTGCGGACGATGGCTGGTCT
CCATGGTCCGAGTGGACCTCCTGTTCTACGAGCTGTGGCAATGGAATTCA
GCAGCGCGGCCGCTCCTGCGATAGCCTCAACAACCGATGTGAGGGCTCCT
CGGTCCAGACACGGACCTGCCACATTCAGGAGTGTGACAAAAGATTTAAA
CAGGATGGTGGCTGGAGCCACTGGTCCCCGTGGTCATCTTGTTCTGTGAC
ATGTGGTGATGGTGTGATCACAAGGATCCGGCTCTGCAACTCTCCCAGCC
CCCAGATGAATGGGAAACCCTGTGAAGGCGAAGCGCGGGAGACCAAAGCC
TGCAAGAAAGACGCCTGCCCCATCAATGGAGGCTGGGGTCCTTGGTCACC
ATGGGACATCTGTTCTGTCACCTGTGGAGGAGGGGTACAGAAACGTAGTC
GTCTCTGCGTCGACTCTAGAATGACTGAAGAGAACAAAGAGTTGGCCAA
TGAGCTGAGGCGGCCTCCCCTATGCTATCACAACGGAGTTCAGTACAGAA
ATAACGAGGAATGGACTGTTGATAGCTGCACTGAGTGTCACTGTCAGAAC
TCAGTTACCATCTGCAAAAAGGTGTCCTGCCCCATCATGCCCTGCTCCAA
TGCCACAGTTCCTGATGGAGAATGCTGTCCTCGCTGTTGGCCCAGCGACT
CTGCGGACGATGGCTGGTCTCCATGGTCCGAGTGGACCTCCTGTTCTACG
 
AGCTGTGGCAATGGAATTCAGCAGCGGCCGCTCCTGCGATAGCCTCAA
CAACCGATGTGAGGGCTCCTCGGTCCAGACACGGACCTGCCACATTCAGG
AGTGTGACAAAAGATTTAAACAGGATGGTGGCTGGAGCCACTGGTCCCCG
TGGTCATCTTGTTCTGTGACATGTGGTGATGGTGTGATCACAAGGATCCG
GCTCTGCAACTCTCCCAGCCCCCAGATGAATGGGAAACCCTGTGAAGGCG
AAGCGCGGGAGACCAAAGCCTGCAAGAAAGACGCCTGCCCCATCAATGGA
GGCTGGGGTCCTTGGTCACCATGGGACATCTGTTCTGTCACCTGTGGAGG
AGGGGTACAGAAACGTAGTCGTCTCTAA (SEQ ID NO:4);
 laminin peptide having the following amino acid sequence: MYIGSR (SEQ ID NO:5), which is encoded by the following DNA sequence (the *Sal*I sites are underlined, and the stop codon is in bold): GTCGACATGTATATTGGTTCTCGTTAAGTCGAC (SEQ ID NO:6); a concatamer of the laminin sequence having the following amino acid sequence (the intervening sequences are underlined): MYIGSRGKSYIGSRGKSYIGSRGKS (SEQ ID NO:7), which is encoded by the following DNA sequence (the *Sal*I sites are underlined, and the intervening sequences are in bold): 
GTCGACATGTATATTGGTTCTCGTGGTAAAAGATATATTGGTTCTCGTGGTAA
AAGATATATTGGTTCTCGTGGTAAAAGATAAGTCGACC (SEQ ID NO:8);
 a peptide from platelet factor-4 having the following amino acid sequence: MLYKKIIKKLLES (SEQ ID NO:9), which is encoded by the following DNA sequence (the *Sal*I sites are underlined): 
GTCGACATGCTTTATAAGAAGATCATCAAGAAGCTTCTTGAGAGTTAAGTCGA
C (SEQ ID NO:10);
 a concatamer of the platelet factor-4 peptide having the following amino acid sequence (the intervening sequences are underlined): 
MLYKKIIKKLLESGKSLYKKIIKKLLESGKSLYKKIIKKLLESGKS (SEQ
ID NO:11),
 which is encoded by the following DNA sequence (the *Sal*I sites are underlined, and the intervening sequences are in bold): 
GTCGACATGCTTTATAAGAAGATCATCAAGAAGCTTCTTGAGAGTGGTAAAAG
ACTTTATAAGAAGATCATCAAGAAGCTTCTTGAGAGTGGTAAAAGACTTTATA
AGAAGATCATCAAGAAGCTTCTTGAGAGTGGTAAAAGATAAGTCGAC (SEQ
ID NO:12);
 somatostatin inhibitor having the following amino acid sequence: MFCYWKVCW (SEQ ID NO:13), which is encoded by the following DNA sequence (the *Sal*I sites are underlined): 
GTCGACATGTTCTTGTATTGGAAGGGATTGTGGTAAGTCGAC (SEQ ID
NO:14);
 a concatamer of somatostatin inhibitor having the following amino acid sequence (the intervening sequences are underlined): MFCYWKVCWGKSFCYWKVCWGKSFCYWKVCWGKS (SEQ ID NO:15), which is encoded by the following DNA sequence (the *Sal*I sites are underlined, and the intervening sequences are in bold): 
GTCGACATGTTCTTGTATTGGAAGGGATTGTGGGGTAAAAGATTCTTGTATTG
GAAGGGATTGTGGGGTAAAAGATTCTTGTATTGGAAGGGATTGTGGGGTAAAA
GATAAGTCGAC (SEQ ID NO:16);
 fibronectin inhibitor having the following amino acid sequence: MGRGD (SEQ ID NO:17), which is encoded by the following DNA sequence (the *Sal*I sites are underlined): 
GTCGACATGTCTTTGTCTTGGAAGACTTTGACTTAAGTCGAC (SEQ ID
NO:18);
 a concatamer of fibronectin inhibitor having the following amino acid sequence (the intervening sequences are underlined): MGRGDGKSGRGDGKSGRGDGKS (SEQ ID NO:19); which is encoded by the following DNA sequence (the *Sal*I sites are underlined, and the intervening sequences are in bold): 
GTCGACATGGGTCGTGGTGATGGTAAAAGAGGTCGTGGTGATGGTAAAAGA
GGTCGTGGTGATGGTAAAAGATAAGTCGAC (SEQ ID NO:20);
 angiostatin having the following amino acid sequence: 
MVYLSECKTGIGNGYRGTMSRTKSGVACQKWGATFPHVPNYSPSTHPNEGLEE
N
YCRNPDNDEQGPWCYTTDPDKRYDYCNIPECEEECMYCSGEKYEGKISKTMSG
LDCQAWDSQSPHAHGYIPAKFPSKNLKMNYCHNPDGEPRPWCFTTDPTKRWEY
C
DIPRCTTPPPPPSPTYQCLKGRGENYRGTVSVTVSGKTCQRWSEQTPHRHNRT
P
ENFPCKNLEENYCRNPDGETAPWCYTTDSQLRWEYCEIPSCESSASPDQSDSS
V PPEEQTPVVQECYQSDGQSYRGTSSTTITGKKCQSEQTPHR
(SEQ ID NO:21),
 which is encoded by the following DNA sequence (the *Sal*I sites are underlined): 
GTCGACATGGTGTATCTGTCAGAATGTAAGACCGGCATCGGCAACGGC
TACAGAGGAACCATGTCCAGGACAAAGAGTGGTGTTGCCTGTCAAAAG
TGGGGTGCCACGTTCCCCCACGTACCCAACTACTCTCCCAGTACACAT
CCCAATGAGGGACTAGAAGAGAACTACTGTAGGAACCCAGACAATGAT
GAACAAGGGCCTTGGTGCTACACTACAGATCCGGACAAGAGATATGAC
TACTGCAACATTCCTGAATGTGAAGAGGAATGCATGTACTGCAGTGGA
GAAAAGTATGAGGGCAAAATCTCCAAGACCATGTCTGGACTTGACTGC
CAGGCCTGGGATTCTCAGAGCCCACATGCTCATGGATACATCCCTGCC
AAATTTCCAAGCAAGAACCTGAAGATGAATTATTGCCACAACCCTGAC
GGGGAGCCAAGGCCCTGGTGCTTCACAACAGACCCCACCAAACGCTGG
GAATACTGTGACATCCCCCGCTGCACAACACCCCCGCCCCCACCCAGC
CCAACCTACCAATGTCTGAAAGGAAGAGGTGAAAATTACCGAGGGACC
GTGTCTGTCACCGTGTCTGGGAAAACCTGTCAGCGCTGGAGTGAGCAA
ACCCCTCATAGGTGA  GTCGAC (SEQ ID NO:22),
 a concatamer of angiostatin having the following amino acid sequence (the intervening sequences are underlined): 
MVYLSECKTGIGNGYRGTMSRTKSGVACQKWGATFPHVPNYSPSTHPNEGLE
ENYCRNPDNDEQGPWCYTTDPDKRYDYCNIPECEEECMYCSGEKYEGKISKTM
SGLDCQAWDSQSPHAHGYIPAKFPSKNLKMNYCHNPDGEPRPWCFTTDPTKRW
EYCDIPRCTTPPPPPSPTYQCLKGRGENYRGTVSVTVSGKTCQRWSEQTPHRH
NRTPENFPCKNLEENYCRNPDGETAPWCYTTDSQLRWEYCEIPSCESSASPDQ
 
SDSSVPPEEQTPVVQECYQSDGQSYRGTSSTTITGKKCQSEQTPHRGKSMVYL
SECKTGIGNGYRGTMSRTKSGVACQKWGATFPHVPNYSPSTHPNEGLEENYCR
NPDNDEQGPWCYTTDPDKRYDYCNIPECEEECMYCSGEKYEGKISKTMSGLDC
QAWDSQSPHAHGYIPAKFPSKNLKMNYCHNPDGEPRPWCFTTDPTKRWEYCDI
PRCTTPPPPPSPTYQCLKGRGENYRGTVSVTVSGKTCQRWSEQTPHRHNRTPE
NFPCKNLEENYCRNPDGETAPWCYTTDSQLRWEYCEIPSCESSASPDQSDSSV
PPEEQTPVVQECYQSDGQSYRGTSSTTITGKKCQSEQTPHR(SEQ ID No
:23),
 which is encoded by the following DNA sequence (the *Sal*I sites are underlined, and the intervening sequences are in bold): 
GTCGACATGGTGTATCTGTCAGAATGTAAGACCGGCATCGGCAACGGCTACA
GAGGAACCATGTCCAGGACAAAGAGTGGTGTTGCCTGTCAAAAGTGGGGTGC
CACGTTCCCCCACGTACCCAACTACTCTCCCAGTACACATCCCAATGAGGGA
CTAGAAGAGAACTACTGTAGGAACCCAGACAATGATGAACAAGGGCCTTGGT
GCTACACTACAGATCCGGACAAGAGATATGACTACTGCAACATTCCTGAATG
TGAAGAGGAATGCATGTACTGCAGTGGAGAAAAGTATGAGGGCAAAATCTCC
AAGACCATGTCTGGACTTGACTGCCAGGCCTGGGATTCTCAGAGCCCACATG
CTCATGGATACATCCCTGCCAAATTTCCAAGCAAGAACCTGAAGATGAATTA
TTGCCACAACCCTGACGGGGAGCCAAGGCCCTGGTGCTTCACAACAGACCCC
ACCAAACGCTGGGAATACTGTGACATCCCCCGCTGCACAACACCCCCGCCCC
CACCCAGCCCAACCTACCAATGTCTGAAAGGAAGAGGTGAAAATTACCGAGG
GACCGTGTCTGTCACCGTGTCTGGGAAAACCTGTCAGCGCTGGAGTGAGCAA
ACCCCTCATAGGGGTAAAAGAATGGTGTATCTGTCAGAATGTAAGACCGGCA
TCGGCAACGGCTACAGAGGAACCATGTCCAGGACAAAGAGTGGTGTTGCCTG
TCAAAAGTGGGGTGCCACGTTCCCCCACGTACCCAACTACTCTCCCAGTACA
CATCCCAATGAGGGACTAGAAGAGAACTACTGTAGGAACCCAGACAATGATG
AACAAGGGCCTTGGTGCTACACTACAGATCCGGACAAGAGATATGACTACTG
CAACATTCCTGAATGTGAAGAGGAATGCATGTACTGCAGTGGAGAAAAGTAT
GAGGGCAAAATCTCCAAGACCATGTCTGGACTTGACTGCCAGGCCTGGGATT
CTCAGAGCCCACATGCTCATGGATACATCCCTGCCAAATTTCCAAGCAAGAA
CCTGAAGATGAATTATTGCCACAACCCTGACGGGGAGCCAAGGCCCTGGTGCT
TCACAACAGACCCCACCAAACGCTGGGAATACTGTGACATCCCCCGCTGCACA
ACACCCCCGCCCCCACCCAGCCCAACCTACCAATGTCTGAAAGGAAGAGGTGA
AAATTACCGAGGGACCGTGTCTGTCACCGTGTCTGGGAAAACCTGTCAGCGCT
GGAGTGAGCAA ACCCCTCATAGGTGA  GTCGAC  (SEQ ID NO:24);
 prolactin having the following amino acid sequence: MLP 
MLPICPGGAARCQVTLRDLFDRAVVLSHYIHNLSSEMFSEFDKRYTHGRGFI
TKAINSCHTSSLATPEDKEQAQQMNQKDFLSLIVSILRSWNEPLYHLVTEVR
GMQEAPEAILSKAVEIEEQTK  (SEQ ID NO:25),
 which is encoded by the following DNA sequence: 
ATGTTGCCCATCTGTCCCGGCGGGGCTGCCCGATGCCAGGTGACCCTTCGAG
ACCTGTTTGACCGCGCCGTCGTCCTGTCCCACTACATCCATAACCTCTCCTC
AGAAATGTTCAGCGAATTCGATAAACGGTATACCCATGGCCGGGGGTTCATT
ACCAAGGCCATCAACAGCTGCCACACTTCTTCCCTTGCCACCCCCGAAGACA
AGGAGCAAGCCCAACAGATGAATCAAAAAGACTTTCTGAGCCTGATAGTCAG
CATATTGCGATCCTGGAATGAGCCTCTGTATCATCTGGTCACGGAAGTACGT
GGTATGCAAGAAGCCCCGGAGGCTATCCTATCCAAAGCTGTAGAGATTGAGG
AGCAAACCTAA (SEQ ID NO:26);
 and a concatamer of prolactin having the following amino acid sequence (the intervening sequences are underlined): 
MLPICPGGAARCQVTLRDLFDRAVVLSHYIHNLSSEMFSEFDKRYTHGRGFIT
KAINSCHTSSLATPEDKEQAQQMNQKDFLSLIVSILRSWNEPLYHLVTEVRGM
QEAPEAILSKAVEIEEQTKGKSMLPICPGGAARCQVTLRDLFDRAVVLSHYIH
NLSSEMFSEFDKRYTHGRGFITKAINSCHTSSLATPEDKEQAQQMNQKDFLSL
IVSILRSWNEPLYHLVTEVRGMQEAPEAILSKAVEIEEQTK (SEQ ID
NO:27),
 which is encoded by the following DNA sequence (the intervening sequences are in bold):

ATGTTGCCCATCTGTCCCGGCGGGGCTGCCCGATGCCAGGTGACCCTTCGAG
ACCTGTTTGACCGCGCCGTCGTCCTGTCCCACTACATCCATAACCTCTCCTC
AGAAATGTTCAGCGAATTCGATAAACGGTATACCCATGGCCGGGGGTTCATT
ACCAAGGCCATCAACAGCTGCCACACTTCTTCCCTTGCCACCCCCGAAGACA
AGGAGCAAGCCCAACAGATGAATCAAAAAGACTTTCTGAGCCTGATAGTCAG
CATATTGCGATCCTGGAATGAGCCTCTGTATCCTGGTCACGGAAGTACGT
GGTATGCAAGAAGCCCCGGAGGCTATCCTATCCAAAGCTGTAGAGATTGAGG
AGCAAACCGGTAAAAGAATGTTGCCCATCTGTCCCGGCGGGGCTGCCCGATGC
CAGGTGACCCTTCGAGACCTGTTTGACCGCGCCGTCGTCCTGTCCCACTACAT
CCATAACCTCTCCTCAGAAATGTTCAGCGAATTCGATAAACGGTATACCCATG
GCCGGGGGTTCATTACCAAGGCCATCAACAGCTGCCACACTTCTTCCCTTGCC
ACCCCCGAAGACAAGGAGCAAGCCCAACAGATGAATCAAAAAGACTTTCTGAG
CCTGATAGTCAGCATATTGCGATCCTGGAATGAGCCTCTGTATCATCTGGTCA

CGGAAGTACGTGGTATGCAAGAAGCCCCGGAGGCTATCCTATCCAAAGCTGTA
GAGATTGAGGAGCAAACCTAA (SEQ ID NO:28)

Increase efficacy will occur with concatamers of the anti-angiogenic genes. This will increase the anti-angiogenic dosage level without changing the amount of vector necessary to deliver these genes. Similar to concatamers, a plasmid with two or more promoters, a plasmid with the IRES sequence (internal ribosomal entry site) between two sequences, and an antiangiogenic peptide with a secretory sequence will increase the delivery of genes to the therapeutic target without markedly increasing the DNA concentration. With regards to the concatamers, the concatamers can extend up to approximately 4400 bases in length (the coding region of a large protein), and the number of concatamers possible will depend on the number of bases of a single anti-angiogenic unit.

For fibronectin, the range of concatamers would be about 2 to 66. Although, the maximum number of anti-angiogenic units for the TSPf is about 6, one can increase this concatameric number by deleting the sequences that do not have any anti-angiogenic effects, such as shown below: 
ATG(CTGAGGCGGCCTCCCCTATGCTATCACAACGGAGTTCAGTACAGAAATA
ACGGTAAAAGATCCCCGTGGTCATCTTGTTCTGTGACATGTGGTGATGGTGTG
ATGGTAAAAGAAGTGGTACCCTGTAGACAAGACAGTGGACACCTCCTCCCCAT
)nTAA (SEQ ID NO:29),
 where the corresponding amino acid sequence is: 
M(LRRPPLCYHNGVQYRNNEEWTVDSGKSSPWSSCSVTCGDGVITRIGKSSPW
DICSVTCGGGV)n (SEQ ID NO:30),
 and wherein n in an integer of from 2 to 24. In a similar manner, the concatameric number of the platelet factor-4 peptide, somatostatin inhibitor, angiostatin, and prolactin can be increased.

Since more than one anti-angiogenic pathway exists, concatamers consisting of two or more types of inhibitor are believed to be more effective than the homogenous concatamers. For example, heterogeneous concatamers of TSPI and the fibronectin inhibitors can be inserted into the same vector. An example of a heterogenous concatamer useful is present invention is as follows: 
ATG(CTGAGGCGGCCTCCCCTATGCTATCACAACGGAGTTCAGTACAGAAATA
ACGGTAAAAGATCCCCGTGGTCATCTTGTTCTGTGACATGTGGTGATGGTGTG
ATGGTAAAAGAAGTGGTACCCTGTAGACAAGACAGTGGACACCTCCTCCCCAT
)x(TATATTGGTTCTCGTGGTAAAAGA)yTAA (SEQ ID NO:31).
 The first parenthetical represents the nucleotide sequence of TSPf, whereas the second parenthetical represents the anti-angiogenic fragment isolated from fibronectin, wherein x and y represent the number of repeats of TSPI and fibronectin, respectively. Again, the number of bases delineated by the summation of x + y will generally not exceed 4400 bases.

The above heterogeneous concatamers need not be limited to only anti-angiogenic peptides. For example, the protein angiostatin or the large polypeptide fragment of prolactin can be modified with with the above-mentioned genes which encode anti-angiogenic pepetides. Again, the concatameric number will vary depending on the number of nucleotide bases of the unit angiogenic inhibitor. In this concatomer of large and small anti-angiogenic inhibitors, the ratio of of large to small inhibitors is 0.1 to 0.9, preferably 1:1.

A translational start signal Met, has been included in all of the above peptides; and a transcriptional stop codon (TAA) has been included in all of the above DNA sequences.

The *Sal*I sites indicated in the above-sequences are a useful cloning tool for insertion of the DNA into BAP vector, which is known to useful in expressing proteins efficiently *in vivo* from the β-actin promoter (Ray et al, *Genes Dev.*, 5:2265-2273 (1991)). Other restriction sites can be incorporated into the DNA for cloning into other vectors.

Other useful vectors for gene therapy which can be employed in the present invention include plasmids with a simian viral promoter, e.g., pZeoSV (Invitrogen); or the CMV promoter, e.g., pcDNA3, pRc/CMV or pcDNA1 (Invitrogen). Plasmids with a CMV promoter may contain an intron 5' of the multiple cloning site (Zhu et al, *supra).* Plasmids containing the BGH terminator instead of the viral SV40 polyA terminator, e.g., pcDNA3, pRc/CMV, pRc/RSV (Norman et al, IBC's 5th Annual Meeting (1995); and Invitrogen vectors), can also be employed in the present invention so as to increase the expression of the tumor suppressor gene and the anti-angiogenic peptide in cells. As stated previously, a vector containing two or more promoters will greatly enhance the therapeutic efficacy. Vectors containing the IRES sequence which allows the translation of two different coding genes to occur from one mRNA transcript will also significantly increase the efficacy of the therapy.

Expression of the DNA encoding the tumor suppressor protein and the DNA encoding the anti-angiogenic peptide can be achieved using a variety of promoters, and the particular promoter employed is not critical to the present invention. For example, the promoter can be a generalized promoter, such as the β-actin promoter, a simian viral promoter, or the CMV promoter, or a tissue specific promoter, such as the α-fetal protein promoter which is specific for liver (Kaneko et al, *Cancer Res.,* 55:5283-5287 (1995), the tyrosinase promoter which is specific for melanoma cells (Hughes et al, *Cancer Res.,* 55:3339-3345 (1995); or the enolase promoter which is specific for neurons (Andersen et al, *Cell. Molec. Neurobiol*., 13:503-515 (1993)).

The particular amount of DNA included in the cationic liposomes of the present invention is not critical. Generally, the amount of total DNA is in the range of about 0.005 to 0.32 µg/pM of liposome, preferably 0.045 to 0.08 µg/pM of liposome.

The DNA encoding a tumor suppressor gene is generally present in an amount of from 0.0025 to 0.16 µg/pM of liposome, preferably 0.028 to 0.04 µg/pM of liposome. The DNA encoding an anti-angiogenic peptide is generally present in an amount of from 0.0025 to 0.16 µg/pM of liposome, preferably 0.028 to 0.04 µg/pM of liposome.

The mole ratio of the DNA encoding the tumor suppressor gene to the DNA encoding the anti-angiogenic peptide is not critical to the present invention. Generally, the mole ratio is between 1:5 to 5:1, preferably about 1 to 1.

The DNA encoding the tumor suppressor gene and the anti-angiogenic peptide may be contained on the same vector, or on separate vectors.

Cationic liposomes are prepared similarly to other liposomes. In brief, the cationic lipid with/or without DOPE are dissolved in a solvent, e.g., chloroform. The lipids are then dried in a round bottom flask overnight on a rotary evaporator. The resulting lipids are then hydrated with sterile water over a 1 hr period so form large mutilamellar vesicle liposomes. To decrease the size of the liposomes, one may sonicate or pass the liposomes back and forth through a polycarbonate membrane. The DNA is then added to a solution containing the liposomes after their formation.

In another embodiment, the above-described objects of the present invention have been met by a method for inhibiting tumor growth in a subject comprising administering to a tumor-bearing subject a cationic liposome:DNA complex comprising DNA encoding a tumor suppressor gene and DNA encoding an anti-angiogenic peptide.

In a preferred embodiment the cationic liposome:DNA complex additionally comprises DNA encoding a tumor suppressor protein.

In a further preferred embodiment the cationic polymer : DNA complex additionally comprises DNA encoding a tumor suppressor protein.
In a further preferred embodiment the cationic liposomes in the cationic liposome:DNA complex are comprised of one cationic lipid (i.e.- 1,2-dioleolyl-sn-glycero-3-ethyl-phosphocholine, 1,2-dimyristoyl-sn-glycero-3-ethylphosphocholine, and 2,3-diol-eyloxy)propyl-N,N,N- tri-methyl-ammonium chloride) and may also be comprised of polyethylene glycol (i.e., a pegylated lipid-1,2-diacy-sn-glycero-3-phospho-ethanolamine-N-Poly(ethylene glycol) 2000) and a targeted ligand (the amino acid peptide RGD, ferritin, or antibodies targeted toward to HER2).

In a further preferred embodiment of the inventionsaid cationic liposomes in said complexes are comprised of one cationic polymer polyethyleneimine, polylysine, polyhisitidine, and Superfect) and may also be comprised of polyethylene glycol and a targeted l ligand (the amino acid peptide RGD, ferritin, or antibodies targeted toward to HER2, CEA).

In a further preferred embodiment said tumor suppressor protein in said complexes is selected from the group consisting of the p53, the p21 and the rb.

In a further preferred embodiment said tumor suppressor protein in the said complexes is p53.

In a further preferred embodiment of the invention said anti-angiogenic peptide in the said complexes is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

In a further preferred embodiment the said DNA encoding an anti-angiogenic peptide used in the said cationic liposome:DNA complexe is present in an amount of from 0.0025 to 0.16 µg/pM of liposome.

In a further preferred embodiment the said DNA encoding an anti-angiogenic peptide in the said cationic polymer:DNA complex is present in an amount of from 0.016 to 0.33 µg/µg of polymer.

In a further preferred embodiment the said DNA encoding a tumor suppressor protein in the said complexes is present in an amount of from 0.0025 to 0.16 µg/pM.

In a further preferred embodiment of the present invention said DNA encoding a tumor suppressor protein in the said complexes is present in an amount of from 0.016 to 0.33 µg/pM.

A further embodiment of the present invention is the provision of the use of a cationic polymer:DNA complex comprising DNA encoding an anti-angiogenic peptide for the production of a medicament for inhibiting tumor growth in a subject which preferably comprises administering the same to a tumor-bearing subject.

In a further preferred embodiment of the present invention the said complex in the said use additionally comprises DNA encoding a tumor suppressor protein.

In a further preferred embodiment of the present invention the said cationic liposome in the said use is (i.e.-1,2-dioleolyl-sn-glycero-3-ethylphosphocholine, 1,2-dimyristoyl-sn-glycero-3-ethylphosphocholine, and 2,3-diol-eyloxy)propyl- N,N,N-trimethylammonium chloride and may also be comprised of polyethylene glycol (i.e., a pegylated lipid-1,2 -diacy-sn-glycero-3-phosphoethanolamine-N-[Poly-(ethylene glycol) 2000) and a targeted ligand (the amino acid peptide RGD, ferritin, or antibodies targeted toward to HER2).

In a further preferred embodiment of the present invention the said cationic polymer in the said use is (*i.e.* (polyethylimine Polycat57, polylysine, polyhisitidine, and Superfect) and may also be comprised of polyethylene glycol and a targeted ligand (the amino acid peptide RGD, ferritin, or antibodies targeted toward to HER2).

In a further preferred embodiment of the present invention the said tumor suppressor protein used in the said cationic polymer is selected from the group consisting of p53, the p21 and the rb.

In a further preferred embodiment of the present invention the said tumor suppressor protein used in the said cationic polymer is p53.

In a further preferred embodiment the said anti-angiogenic peptide used for the said cationic polymer is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

In a further preferred embodiment of the present invention the said DNA encoding an anti-angiogenic peptide used in the cationic complex is present in an amount from 0.0025 to 0.16 µg/pM of liposome or 0.016 to 0.33 µg/µg of polymer.

In a further preferred embodiment of the present invention the said DNA encoding a tumor suppressor protein used in the said complex is present in an amount of from 0.0025 to 0.16 µg/pM of liposome or 0.016 to 0.33 µg/µg of polymer.

In a further embodiment of the present invention the said cationic polymer:DNA complex comprising a plasmid with one or more promoters expressing either the same gene product (SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30) or combinations of these gene products.

In a further embodiment of the present invention the cationic liposome:DNA complex comprising a plas-mid with an intervening internal ribosomal entry site sequence between two genes (SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, and SEQ ID NO:29) that are either the same or different.

In a further preferred embodiment of the present invention the said anti-angiogenic protein in the said cationic liposome:DNA complex or the said cationic polymer:DANN complex is secretory from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

The particular type of tumor which can be treated in the present invention is not critical thereto. Examples of tumors which can be treated in accordance with the present invention include solid tumors, e.g., lung, colon, brain, breast and melanoma tumors. All of these tumors are very dependent on blood supply to sustain their growth.

The particular mode of administering the cationic liposome:DNA complex of the present invention is not critical thereto. Examples of such modes include intravenous, subcutaneous and intratumoral injections. Intravenous injection is the preferred administration mode since there is better distribution to the developing blood vessels of the tumor.

The amount of cationic liposome:DNA complex to be administered will vary depending upon the age, weight, sex of the subject, as well as the tumor volume and rate of tumor growth in the subject. Generally, the amount to be administered will be about 5 to 60 µg, preferably about 9 to 16 µg.

The following examples are provided for illustrative purposes only, and are in no way intended to limit the scope of the present invention.

### EXAMPLE 1

### Production of DNA Vectors

### A. TSPI Vector

The coding region of the TSPI gene is well-known (GB Accession code-X14787). The TSPI gene was inserted into the *Xba*I site of BAP vector (Ray et al, *supra),* so as to give rise to TSPI vector, wherein expression of the TSPI gene is controlled by the β-actin promoter.

More specifically, TSPI cDNA and Bluescript plasmid (Promega) were digested with *Hind*I and *Xba*I, and then the TSPI cDNA was ligated into Bluescript. Next, Bluescript containing the TSP cDNA and BAP vector were digested with *Sal*I and *Bam*HI, and TSPI cDNA inserted in the *Xba*I site of BAP vector. The correct orientation of the TSPI gene in BAP vector was confirmed by DNA sequencing.

### B. TSPf Vector

TSPf vector is a vector containing a DNA fragment of the TSPI gene which has the two anti-angiogenic domains (nucleotides 992-1650) (Tolsma et al, *supra),* and a start codon and a stop codon.

The DNA fragment was prepared by PCR using thrombospondin I cDNA as template, and 100 pmoles of each of the following primers 5'-TAGGTCTAGA**ATG**ACTGAAGAGAACAAAGAG-3' (SEQ ID NO:24); and 5'-ATGGTCTAGA**TTA**GAGACGACTACGTTTCTG-3' (SEQ ID NO:25) so as to amplify nucleotides 1013 to 1650 of the TSPI gene. Both primers contain *Xba*I sites (underlined), the first primer contains an ATG start codon (in bold), and the second primer contains a TTA stop codon (in bold).

The resulting 638 base pair fragment of the TSPI gene (hereinafter "TSPf") encodes the peptides that are known to be angiogenic inhibitors (Tolsma et al, *supra*).

After amplification, the DNA fragment was purified, digested with *Xba*I, and the digested fragment inserted into the *Xba*I site of BAP vector such that the expression of the TSPf gene was controlled by the β-actin promoter (Ray et al, *supra;* and Lesoon-Wood et al, *Human Gene Ther.,* 6:395-405 (1995)). The correct orientation of the fragment in BAP vector was verified by digestion with *Bam*HI, and confirmed by DNA sequencing.

### C. p53 Vector

The coding sequence of the p53 gene was cut from plasmid p1SVhp53c62 (Zakut-Houri et al, *EMBO J.,* 4:1251-1255 (1985)) with *Xba*I, and inserted into the multiple cloning sites of pGEM3Z vector (Promega, Madison, WI). Digestion of the resulting vector with *Sal*I and *Bam*HI generated a 1900 bp fragment that was then inserted into the *Sal*I and *Bam*HI sites of BAP vector such that expression of the p53 gene was controlled by the β-actin promoter. The correct orientation of the p53 gene in BAP vector was confirmed by DNA sequencing.

### EXAMPLE 2

### Preparation of Cationic Liposome:DNA Complexes

A DOTMA:DOPE liposome mixture is known to efficiently transfect endothelial cells *in vitro* (Tilkins et al, *Focus,* 16:117-119 (1994)). Accordingly, liposome:DNA complexes were prepared using DOTMA:DOPE, in a 1:1 ratio, essentially as described by Debs et al, *J. Biol. Chem*., 265:10189-10192 (1990). Similar liposomes preparations can be prepared by mixing, at a 1:1 ratio, DOPE with other cationic lipids, such as, 1,2-dioleolyl-sn-glycero-3-ethylphophocholine, and 1,2-dimyristoyl-sn-glycero-3-ethylphophocholine.

More specifically, a mixture of 400 nmoles of the DOTMA and DOPE were dried overnight on a rotary evaporator. Then, the lipids were rehydrated with 1.5 ml of water for 2 hrs. Next, the milky liposome preparation was sonicated with a bath sonicator until clear. The resulting liposome preparation was then passed through a 50 nm polycarbonate filter between 15 to 20 times with a LipsoFast-Basic extruder (Avestin, Ottawa, On).

The DNA employed was either (1) empty BAP vector; (2) TSPI vector alone; (3) TSPf vector alone; (4) p53 vector alone; (5) p53 vector + TSPI vector; or (6) p53 vector + TSPf vector.

The DNA was prepared with the maxi Qiagen kits (Qiagen Inc., Chatsworth, Ca), and washed twice in 70% (v/v) ethanol. The DNA was then dialyzed against water for 24 hrs to removed any remaining salt.

About 400 pmols of the liposome preparation was gently mixed with between 18 to 35 µg of total DNA in an Eppendorf tube. This amount in each eppendorf tube was sufficient for two injections. The same amount of DNA was injected in the combination therapies as in the single treatment regimens. For example, if 16 µg of DNA in the combination therapy (8.0 µg of p53 + 8.0 µg of TSPf) was injected into each mouse of one group, then 16 µg of p53 was injected into each mouse of a second group.

### EXAMPLE 3

### Anti-Angiogenic Effect of Cationic Liposome:DNA Complexes

The anti-angiogenic effects of the cationic liposome:DNA complexes obtained in Example 2 were evaluated in mice containing MDA-MB-435 breast cancer tumors (American Type Tissue Culture, Bethesda, MD), which are p53 deficient.

More specifically, after administering the anesthetic, Metofane, to 126 female athymic nude mice (NCI), the mice were injected with 2.0 x 10⁵ MDA-MB-435 tumor cells into the mammary fat pad using a stepper (Tridak) and a 27.5 g needle. Two weeks later, the mice whose tumors grew were divided into various treatment regimens, 18 mice per each regimen. The treatment regimens were as follows: (1) untreated; (2) empty BAP vector; (3) TSPI vector alone; (4) TSPf vector alone; (5) p53 vector alone; (6) p53 vector + TSPI vector; and (7) p53 vector + TSPf vector. The mice received two intravenous injections, the first injection 14 days after the malignant cells had been implanted into the mice, and the second injection 24 days after the malignant cells had been implanted into the mice. The first injection consisted of 200 pmoles of the liposomes complexed with 16 µg of total DNA. The second injection consisted of 200 pmols of the liposomes complexed with 8.0 µg of total DNA. The sizes of the tumors were measured 7 days after the second injection. The results are shown in Table 1 below.

**TABLE 1**

| Anti-tumor Effects of TSPI and TSPf | |
|---|---|
| Putative Anti-tumor Genes | Tumor Size (mm³) |
| Untreated | 113.5±6.41 |
| BAP | 102.9±6.83 |
| TSPI | 103.2±8.96 |
| TSPf | 89.4±11.06 |
| p53 | 80.1±12.7* |
| p53 + TSPI | 82.9±6.95* |
| p53 + TSPf | 53.2±8.37** |

| | |
|---|---|
| * p53 or p53 + TSPI vs. untreated, p<0.05 | |
| ** p53 + TSPf vs. untreated or BAP, p<0.01 | |

As shown in Table 1 above, the p53-treated group was found to be statistically different from the untreated group (p<0.05) after 2 injections. However, the p53 treated group was not statistically different from the empty BAP vector group. This was similar to the results described by Lesoon-Wood et al, *Human Gene Ther.,* 6:395-406 (1995), in which p53 was not statistically different from the empty BAP vector group until after 5 injections.

However, p53 in combination with TSPf reduced tumors more effectively than p53 alone. That is, after just 2 injections of this combination therapy, there was a 35% further reduction in tumor growth compared to p53 alone. The combination group was statistically different from both the untreated and the empty BAP vector groups (p<0.01). Although TSPf by itself was slightly less effective than p53, TSPf was, unexpectedly, substantially more effective than TSPI. In fact, the full length TSPI-treatment group had no more effect than either the empty vector or the untreated groups. This was unexpected for several reasons: 1) both the full length and the fragment of thrombospondin I contained the anti-angiogenic peptide 2) in a previous ex vivo study (Weinstat-Saslow et al, supra), full length thrombospondin I was effective in inhibiting tumor growth, and 3) full length thrombospondin I has a secretory sequence presumably so that the secreted protein can inhibit endothelial proliferation, whereas the thrombospondin I fragment does not contain a secretory sequence.

Regardless of whether there is a secretory sequence, one would predict prior to the present invention that the liposome:antiangiogenic gene would not be an effective antitumor therapy. As taught by Lesoon-Wood et al., the transfection efficiency of the tumor with cationic liposomes was very low. In fact, it could not be quantitated with a primer extension method. We know from the teaching of Weinstat-Saslow et al. that high levels of expressed TSPI in 100% of the tumor cells reduces the tumor growth by only 60% in an *ex vivo* system. Extrapolating from these findings, a relatively high transfection efficiency of 20% with the liposome: antiangiogenic genes would have resulted in a marginal reduction (20%/100% X 60% reduction = 12%) of the tumor. This amount of tumor reduction would not have resulted in statistical differences with the liposome:antiangio-genic gene complexes. A transfection efficiency of the tumor above 10% would have easily been measurable with a variety of techniques including the primer extension method (used by Lesoon-Wood et al.). It has been determined that the transfection efficiency of the tumor is less than 5% with these cationic liposomes.

Hence, it was clearly unobvious that DNA encoding an anti-angiogenic peptide alone would be an effective anti-tumor agent *in vivo,* based upon teachings that an anti-angiogenic peptide is an effective anti-tumor agent (Tolsma et al and Bouck et al), and based upon the teachings that DNA encoding a full-length anti-angiogenic protein is an effective anti-tumor agent *ex vivo* (Weinstat-Saslow et al).

A second experiment was carried out to determine whether the combination therapy of p53 and TSPf was effective at lower dosages, and to confirm that the combination of p53 and TSPf reduced the tumor size considerably more than p53 alone.

More specifically, 36 mice were injected with 2.0 x 10⁵ MDA-MB-435 tumor cells into the mammary fat pad. Two weeks later, the mice whose tumors grew were divided into various treatment regimens, 18 mice per each regimen. The treatment regimens were as follows: (1) empty BAP vector; (2) p53 vector alone, and (3) p53 vector + TSPf vector. The mice were injected intravenously with 200 pmols of the liposomes complexed with 8.0 µg of total DNA. Subsequently, the mice were treated in the same manner with 200 pmols of the liposomes complexed with 12 µg of total DNA for the next 4 injections. Ten days elapsed between each injection. The sizes of the tumors were measured before each injection and 7 days after the last injection. The results are shown in Table 2 below:

**TABLE 2**

| Anti-tumor Effects of p53 and TSPf | |
|---|---|
| Putative Anti-tumor Genes | Tumor Size (mm³) |
| BAP | 855±345 |
| p53 | 616±142 |
| p53 + TSPf | 265±133* |

| | |
|---|---|
| * p53 + TSPf vs. BAP, p<0.02 | |

As shown in Table 2 above, the combination therapy with p53 and TSPf was statistically different from BAP, whereas the p53 alone treatment was not. This experiment confirmed that p53 and TSPf were more effective than p53 alone. Furthermore, a different dosage regimen, without an initial booster dose of 16 µg of DNA as used in the experiment in Table 1, accentuated the difference between the combination treatment and the p53 alone treatments.

In table I, the TSPf treatment group decreased the tumor more than empty vector or untreated groups. However, it was not statistically significant (p=.07). We repeated the experiment after injecting a higher dose of DNA and measured the different treatment groups tumors 10 days after the first treatment.

**Table 3**

| Anti-tumor Effects of TSPf | |
|---|---|
| Putative Anti-tumor Genes | Tumor Size (mm³) |
| Untreated | 80.0±11.2 |
| BAP | 80.4±4.5 |
| TSPf | 50.7±4.8* |

| | |
|---|---|
| * TSPf vs. BAP, p<0.025 | |

At the higher dose of 19 ugs of DNA, the TSPf treatment group was statistically different from either the empty vector or the untreated groups.

In another experiment demonstrating the efficacy of antiangiogenic genes, various antiangiogenic genes were exaimined for their antitumor activity. After administering the anesthetic, Metofane, to 126 female athymic nude mice (NCI), the mice were injected with 3.0 x 10⁵ MDA-MB-435 tumor cells into the mammary fat pad using a stepper (Tridak) and a 27.5 g needle. Two weeks later, the mice whose tumors grew were divided into various treatment regimens, 8 mice per each regimen. The treatment regimens were as follows: (1) BAP vector; (2) TSPf vector alone; (3) laminin peptide vector alone; and (4) angiostatin vector alone. The mice received 4 intravenous injections, the first injection was 10 days after the malignant cells had been implanted into the mice, and the remaining injections were thereafter 10 days apart. The injections consisted of 200 pmoles of the liposomes complexed with 12.5 µg of total DNA. The results are shown in Table 4 below.

**TABLE 4**

| Putative Anti-tumor Genes | Tumor Size (mm³) |
|---|---|
| BAP | 194.7 ± 11.9 |
| TSPf | 135.9 ± 11.9* |
| Laminin peptide | 126.4 ± 8.4* |
| Angiostatin | 95.2± 6.3*^{,}** |

| | |
|---|---|
| * TSPf, Laminin peptide, and Angiostatin vs. BAP, p<0.05 | |
| ** Angiostatin vs. BAP, p<0.01 | |

As shown in Table 4 above, the cationic liposomes containing only DNA encoding various anti-angiogenic peptides (TSPf, laminin peptide and angiostatin) significantly inhibited tumor growth.

Next, MCF7 cells (American Type Tissue Culture, Bethesda, MD), which are a breast cancer cell line with two normal p53 alleles, were evaluated as described above except that 4.0 x 10⁶ cells were injected into the mice; and the third injection contained 12 µg of the DNA. Each injection was 10 days apart. Nine mice were injected with each of the following treatments except for regimen (1), in which 8 mice were treated: (1) untreated; (2) BAP; (3) p53; and (4) p53 + TSPf. The sizes of the tumors were measured 7 days after the third injection. The results are shown in Table 5 below.

**TABLE 5**

| Effect of p53 and TSPf on MCF7s Cells | |
|---|---|
| Putative Anti-tumor Genes | Tumor Size (mm³) |
| Untreated | 124.6±7.3 |
| BAP | 136±16.8 |
| p53 | 83.1±13.6* |
| p53 + TSPf | 69.0±13** |

| | |
|---|---|
| * p53 vs. untreated or BAP, p<0.05 | |
| ** p53 + TSPf vs. untreated or BAP, p<0.01 | |

As shown in Table 5 above, the most effective therapy against MCF7s was p53 and TSPI. The significance level for the p53 + TSPf therapy was greater than for p53 alone when they were compared against either the untreated or the BAP groups.

The above experiment verifies that p53 and TSPfI decreased the MCF7s tumor more than the p53 treated or the untreated groups. 4 X 10⁵ MCF7 cells were injected bilaterally into the mammary fat pads of the 28 nude mice. After two weeks of growth, these mice were randomly divided into four groups: 1)empty vector, 2) p53, 3) p53 + TSPf , and 4) untreated. The mice received one injection of 200 pmoles of liposomes complexed with 14 ugs of DNA, and the tumors from the various treatment groups were measured 10 days after the treatment. The results are shown in Table 6 below.

**Table 6**

| Putative Anti-tumor Genes | Tumor Size (mm³) |
|---|---|
| Empty vector- | 54.7±4.0 |
| p53 | 45.5±5.0 |
| p53 + TSPf | 33.9±3.6* |
| Untreated | 61.9±8.3 |

| | |
|---|---|
| *, p53 + TSPf vs Untreated, p<.025 | |

As shown in Table 6, the additional reduction of the tumor by the combined use of p53 and TSPf (also
in Tables 1, 2, and 4 above) compared to the use of p53 only, suggest that TSPf and p53 have different mechanisms of action. Although this does not preclude that the target of p53 is the vasculature of the tumor, the mechanism of inhibition of the tumor by p53 is not known at present. However, any mechansim of tumor inhibition by p53 and/or thrombospondin I must account for the low transfection efficiency of the tumor. Again, with a liposome complexed to a chloramphenicol acetyltransferase marker, it has been demonstrated that less than 5% of the tumor derived from MDA-MB-435 cells was transfected with the marker gene.

Besides p53 and the antiangiogenic fragment of thrombospondin I, we determined that liposomes complexed to DNA encoding the laminin peptide inhibits tumor growth. More specifically, after administering the anesthetic, Metofane, to 24 female athymic nude mice, the mice were injected with 3.0 x 10⁵ MDA-MB-435 tumor cells into the mammary fat pad using a stepper and a 27.5 g needle. Two weeks later, the mice whose tumors grew were divided into various treatment regimens, 8 mice per each regimen. The treatment regimens were as follows: (1) BAP, (2) laminin, and (3) p53 + laminin. The mice were injected intravenously with 200 pmols of the liposomes complexed with 12.5 mgs of total DNA 6.25 mg of each vector when a combination was used. The mice then received 3 injections, each 10 days apart. The tumors were measured at the time of each injection and at the time of the last injection. The results are shown in Table 7 below.

**TABLE 7**

| Putative Anti-tumor Genes | Tumor Size (mm³) |
|---|---|
| BAP | 345 ± 23.5 |
| Laminin peptide | 280 ± 32.4 |
| Laminin peptide + p53 | 192 ± 10.5* |

| | |
|---|---|
| * BAP vs. Laminin peptide + p53, p<0.05 | |

As shown in Table 7 above, cationinc liposomes containing a combination of DNAs encoding laminin peptide + p53 was unexpectedly more effective in reducing tumor growth than when DNA encoding the anti-angiogenic peptide was used alone. Thus, the addition of a tumor suppressor gene, p53, enhances the anti-tumor effect of the anti-angiogenic peptide.

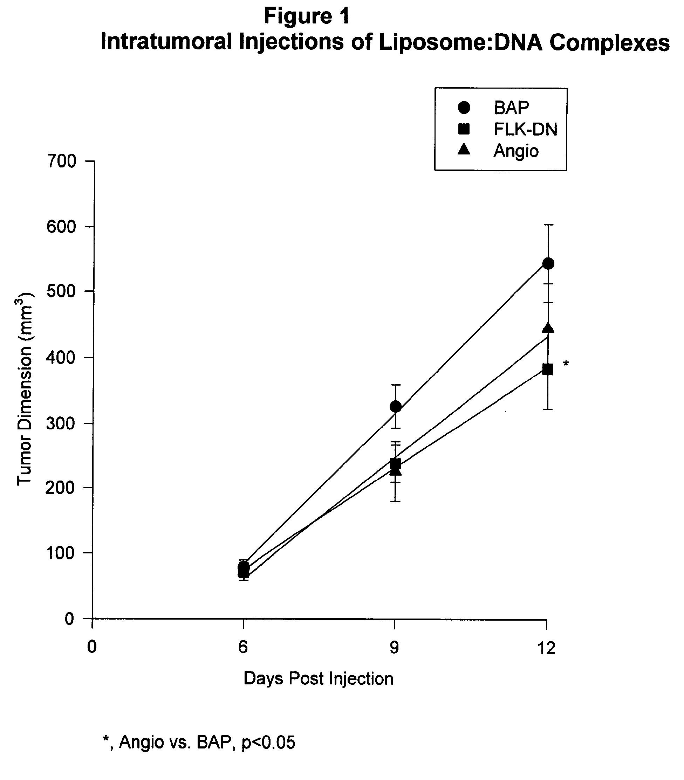

Although intravenous injection are preferred, the method of administration of the liposome:DNA complex is not critical. In figure 1, it was found that intratumoral injections are effective, and it also supports that the therapy is effective against tumors other than breast cancer. In this experiment, 18 mice were injected with 3X10⁵ C6 glioma cells (rat brain tumors) subcutaneously. Six days after the injections of these cells, the mice were separated into 3 groups: 1)BAP, 2)FLK-DN (a dominant negative receptor), and 3) angiostatin. After the second intratumoral injection, there was a statistical difference between the angiostatin and the BAP groups. Thus, this therapy is effective when given intratumorally and is effective as expected tumors other than breast tumors.

It was also found that this liposome: secretory angiostatin construct was more effective that the non-secreted analog. In brief, we injected 24 nude mice with 3X10⁵ MDA-MB-435 cells inserted at the 5 prime end of the their construct. Two weeks later the mice were divided into three groups, they received the following therapies intravenously:1) liposome:BAP, 2) liposome:secreted angiostatin, and 3) liposome:angiostatin. The concentration of DNA injected into the mice was 14.5 ugs. The mice received one injection of the liposome:DNA complex and their tumors were measured 10 days after the injection.

**Table 8**

| Efficacy of Secretory Angiostatin | |
|---|---|
| Therapeutic Genes | Tumor Size (mm3) |
| Angiostatin | 28.8±2.2 |
| Angiostatin-Secretory | 18.6±1.8* |
| BAP | 30.5±3.3 |

| | |
|---|---|
| *,p<0.05, BAP vs. Angiostatin-secretory | |

As seen in table 8, the secretory angiostatin treatment group was much more effective than the empty vector control or the angiostatin treatment group in reducing the size of the tumor. From this experiment, it is evident that a secretory sequence inserted into the 5' portion of the antiangiogenic inhibitor will increase its efficacy.

In vitro assays indicate that cationic polymers will significantly improve the present therapy. When a carrier such as a cationic lipid was used in this *in vitro* assay, the inhibitory effect (of the genes p53, TSPf, and the combination of p53 and TSPf) was marginal whereas another vector, Superfect (a cationic polymer), was much more effective as a carrier. This is because Superfect was 15 times more effective than the cationic liposomes in transfecting endothelial cells with the CAT marker. The cationic liposomes used in this section was DOSPER (Boerhinger), which of the 14 lipids tested gave the best results. Included in this panel of 14 lipids that we tested was lipofectin (BRL) which is a mixture of DOTMA/DOPE that we have used in an *in vivo* study. In brief, we plated 1X10⁶ Huvec cells into each well of a 6 well plate. 25uls of Superfect complexed with 2 ugs of DNA was added to each plate 24 hours after the initial seeding of the cells. 36 hours after the transfection, the cells were lysed and the amount of CAT protein was assayed.

**Table 9**

| Vectors | Activity(DPMs/protein) |
|---|---|
| Cationic liposomes with BAP | 31.1±7.2 |
| Cationic liposomes with CAT | 682±129 |
| Superfect with BAP | 21.4±0.458 |
| Superfect with CAT | 10816±687 |
| p<0.001, Superfect-CAT vs Cationic liposome-CAT | |

This experiment clearly demonstrates that this cationic polymer is a superior in the transfection of endothelial cells, which is a likely target of the therapeutic gene. We have found that Superfect is a better transfection agent than cationic liposomes for many different cell lines. Since Superfect which is a cationic polymers is such an efficient carrier of DNA, this underscores possibility that non-viral carriers as a class of carriers will be effective in decreasing the tumor size. As a result, other non-viral carriers besides liposomes should be included in this patent.

Transfection of Huvec Cells with various inhibitors was as follows. 1.0 x 10⁵ Huvec cells (Clonetics), a human endothelial cell line, were plated into each well of a 6-well plate, and placed in a CO₂ incubator at 37°C. Twenty-four hours later, the cells were transfected with 25 ml of Superfect (Qiagen), a cationic polymer, complexed to 2.0 mg of various DNA vectors, i.e., (1) BAP vector; (2) p53 vector; (3) TSPf vector; and (4) p53 vector + TSPf vector. After the cells were exposed for 2 hours to this complex at 37°C, the media was removed, and replaced with fresh EGM media (Clonetics, Inc.). containing 10% (v/v) fetal calf serum, and 1.0% (w/v) glutamine, and the cells placed in a CO₂ incubator at 37°C. Twenty-four hours later, the cell number in each plate was determined by the 3-(4,5-dimethylthiazol-2-yl)--(3-carboxymethoxyphenyl)- 2-,5-di- phenyltetetra-zolium bromide (MTS) assay described by Butke et al, *J. Immunol. Methods,* 157:233-240 (1993).

The results are shown in **Figure 1** attached hereto dealing with the intratumoral injections of liposome:DNA complexes and its effect on the tumor dimension 6 to 12 days after injection.

As shown in **Figure 1**, it was found that p53, TSPf, and the combination therapy of p53 and TSPf were effective at inhibiting endothelial cells *in vitro.* The combination of p53 and TSPf was the most effective at inhibiting endothelial cells. There was a close correlation between the therapeutic genes reducing the tumor size *in vivo* and their effects on endothelial cell number *in vitro.*

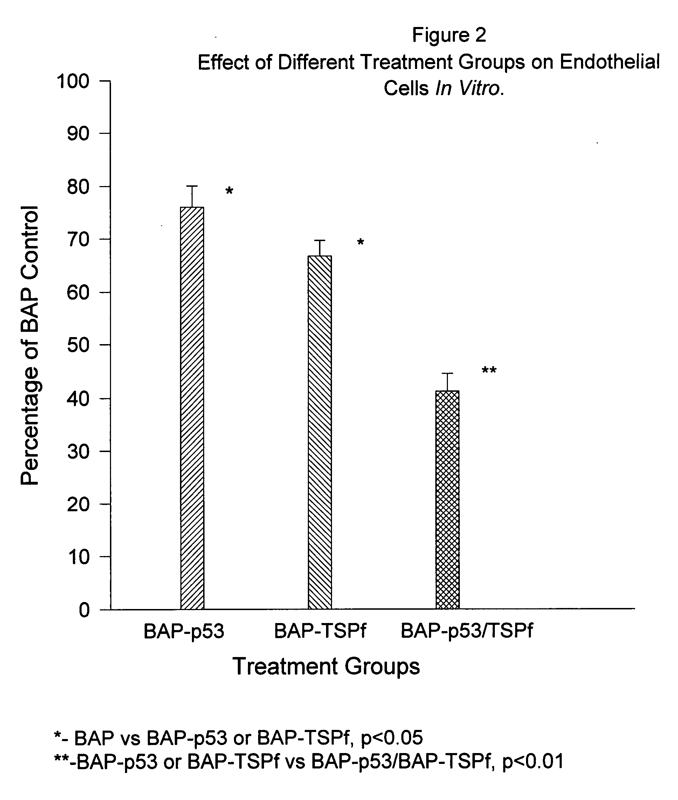

**Figure 2** shows the effect of different treatment groups on endothelial cells in vitro. it was found that the percentage of BAP control is significantly decreased when using BAP-p53 and BAPf-TSPf as tretment groups. A further synergistic decrease is achieved when using BAP-p53/TSPf a the treatment group.

While the invention has been described in detail, and with reference to specific embodiments thereof, it will be apparent to one of ordinary skill in the art that various changes and modifications can be made therein without departing from the spirit and scope thereof.

## Claims

1. A cationic polymer:DNA complex comprising the DNA encoding an antiangiogenic peptide.

2. The complex of Claim 1, wherein said complex additionally comprises DNA encoding a tumor suppressor protein.

3. The complex of Claim 2, wherein said tumor suppressor protein is selected from the group consisting of the p53, the p21 and the rb.

4. The complex of Claim 2, wherein said tumor suppressor protein is p53.

5. The complex of Claim 1, wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

6. The complex of Claims 1 or 2, wherein said DNA encoding an anti-angiogenic peptide is present in an amount of from 0.016 to 0.33 µg/µg of polymer.

7. The complex of Claim 5, wherein said DNA encoding a tumor suppressor protein is present in an amount of from 0.0025 to 0.16 µg/pM.

8. The complex of Claim 5, wherein said DNA encoding a tumor suppressor protein is present in an amount of from 0.016 to 0.33 µg/pM.

9. Use of a cationic polymer:DNA complex comprising DNA encoding an anti-angiogenic peptide for the production of a medicament for inhibiting tumor growth in a subject which preferably comprises administering the same to a tumor-bearing subject.

10. The use of Claim 9, where said complex additionally comprises DNA encoding a tumor suppressor protein.

11. The use of claim 1, wherein said cationic polymer is selected from the group consisting of (polyethylenimine Polycat57, polylysine, polyhisitidine, and Superfect) and may also be comprised of polyethylene glycol and a targeted ligand (the amino acid peptide RGD, ferritin, or antibodies targeted toward to HER2).

12. The use of Claim 10, wherein said tumor suppressor protein is selected from the group consisting of p53, the p21 and the rb.

13. The use of Claim 12, wherein said tumor suppressor protein is p53.

14. The use of Claim 9, wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

15. The use of Claim 9 or Claim 10, wherein said DNA encoding an anti-angiogenic peptide is present in an amount from 0.0025 to 0.16 µg/pM of liposome or 0.016 to 0.33 µg/µg of polymer.

16. The use of Claims 9 or 10, wherein said DNA encoding a tumor suppressor protein is present in an amount of from 0.0025 to 0.16 µg/pM of liposome or 0.016 to 0.33 µg/µg of polymer.

17. A cationic polymer:DNA complex comprising a plasmid with one or more promoters expressing either the same gene product (SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30) or combinations of these gene products.

18. The complex of Claim 1, wherein said anti-angiogenic protein is secretory from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.
